# EUROPEAN PATENT APPLICATION

(11) **EP 2 228 387 A2**
(43) Date of publication of application: **15.09.2010**
(21) Application number: 10154130.8
(22) Date of filing: 17.04.2002
(51) Int. Cl.: C07K 14/47, C07K 5/09, C07K 5/11, C07K 5/103, A61K 38/17, C07K 4/00, C07K 14/00, C07K 7/06, A61K 38/03, A61K 38/06, A61K 38/07, A61K 38/08, A61K 38/16, A61P 25/28, C07K 5/00

(54) **Polypeptides derived from amyloid precursor peptide (app) and their uses**

(30) Priority: 18.04.2001 GB 0109558; 17.08.2001 GB 0120084; 30.11.2001 US 998491; 28.03.2002 GB 0207387
(62) Divisional of application: 02720228.2
(71) Applicant: THE OPEN UNIVERSITY, Milton Keynes MK7 6AA (GB)
(72) Inventor: Mileusnic, Radmila, Milton Keynes MK7 6AA (GB); Rose, Steven Peter Russell, Milton Keynes MK7 6AA (GB)
(74) Representative: Ackroyd, Robert

(57) **Abstract**

The invention provides a compound having formula
X₁-Arg-Xaa-Arg-X₂
in which X₁ and X₂ are up to 30 amino acid residues and Xaa is an amino acid residue. A preferred compound is the tripeptide Arg-Glu-Arg which corresponds to amino acid residues 328 to 330 of human amyloid precursor protein. The invention further provides a derivative of a polypeptide having the formula:
X₁-Arg-Xaa-Arg-X₂
wherein X₁ and X₂, which may be the same or different, each represents from zero to 30 natural or synthetic amino acid residues or derivatives thereof and Xaa represents a natural or synthetic amino acid residue or a derivative thereof, at least one functional group of at least one said amino acid residue or derivative thereof being protected by a protective group. The compounds of the invention are believed to be useful in the treatment of Alzheimer's disease and as cognitive enhancers.

SEQUENCE LISTING

<110> The Open University

<110> Rose, Steven Peter Russell

<110> Mileusnic, Radmila

<120> Polypeptides, derivatives and uses thereof

<130> RA/P304965EP

<140> EP10.........
<141> 2002-04-17

<150> GB 0109558.7
<151> 2001-04-18

<150> GB 0120084.9
<151> 2001-08-17

<150> US 09/998491
<151> 2001-11-30

<150> GB 0207387.2
<151> 2002-03-28

<160> 22

<170> PatentIn Ver. 2.1

<210> 1
<211> 695
<212> PRT
<213> Homo sapiens

<400> 1
   Met Leu Pro Gly Leu Ala Leu Leu Leu Leu Ala Ala Trp Thr Ala Arg
     1               5                  10                  15
   Ala Leu Glu Val Pro Thr Asp Gly Asn Ala Gly Leu Leu Ala Glu Pro
                20                  25                  30
   Gln Ile Ala Met Phe Cys Gly Arg Leu Asn Met His Met Asn Val Gln
            35                  40                  45
   Asn Gly Lys Trp Asp Ser Asp Pro Ser Gly Thr Lys Thr Cys Ile Asp
        50                  55                  60
   Thr Lys Glu Gly Ile Leu Gln Tyr Cys Gln Glu Val Tyr Pro Glu Leu
    65                  70                  75                  80
   Gln Ile Thr Asn Val Val Glu Ala Asn Gln Pro Val Thr Ile Gln Asn
                    85                  90                  95
   Trp Cys Lys Arg Gly Arg Lys Gln Cys Lys Thr His Pro His Phe Val
               100                 105                 110
   Ile Pro Tyr Arg Cys Leu Val Gly Glu Phe Val Ser Asp Ala Leu Leu
           115                 120                 125
   Val Pro Asp Lys Cys Lys Phe Leu His Gln Glu Arg Met Asp Val Cys
       130                 135                 140
   Glu Thr His Leu His Trp His Thr Val Ala Lys Glu Thr Cys Ser Glu
   145                 150                 155                 160
   Lys Ser Thr Asn Leu His Asp Tyr Gly Met Leu Leu Pro Cys Gly Ile
   165 170 175
   Asp Lys Phe Arg Gly Val Glu Phe Val Cys Cys Pro Leu Ala Glu Glu
   180 185 190
   Ser Asp Asn Val Asp Ser Ala Asp Ala Glu Glu Asp Asp Ser Asp Val
   195 200 205
   Trp Trp Gly Gly Ala Asp Thr Asp Tyr Ala Asp Gly Ser Glu Asp Lys
   210 215 220
   Val Val Glu Val Ala Glu Glu Glu Glu Val Ala Glu Val Glu Glu Glu
   225 230 235 240
   Glu Ala Asp Asp Asp Glu Asp Asp Glu Asp Gly Asp Glu Val Glu Glu
   245 250 255
   Glu Ala Glu Glu Pro Tyr Glu Glu Ala Thr Glu Arg Thr Thr Ser Ile
   260 265 270
   Ala Thr Thr Thr Thr Thr Thr Thr Glu Ser Val Glu Glu Val Val Arg
   275 280 285
   Val Pro Thr Thr Ala Ala Ser Thr Pro Asp Ala Val Asp Lys Tyr Leu
   290 295 300
   Glu Thr Pro Gly Asp Glu Asn Glu His Ala His Phe Gln Lys Ala Lys
   305 310 315 320
   Glu Arg Leu Glu Ala Lys His Arg Glu Arg Met Ser Gln Val Met Arg
   325 330 335
   Glu Trp Glu Glu Ala Glu Arg Gln Ala Lys Asn Leu Pro Lys Ala Asp
   340 345 350
   Lys Lys Ala Val Ile Gln His Phe Gln Glu Lys Val Glu Ser Leu Glu
   355 360 365
   Gln Glu Ala Ala Asn Glu Arg Gln Gln Leu Val Glu Thr His Met Ala
   370 375 380
   Arg Val Glu Ala Met Leu Asn Asp Arg Arg Arg Leu Ala Leu Glu Asn
   385 390 395 400
   Tyr Ile Thr Ala Leu Gln Ala Val Pro Pro Arg Pro Arg His Val Phe
   405 410 415
   Asn Met Leu Lys Lys Tyr Val Arg Ala Glu Gln Lys Asp Arg Gln His
   420 425 430
   Thr Leu Lys His Phe Glu His Val Arg Met Val Asp Pro Lys Lys Ala
   435 440 445
   Ala Gln Ile Arg Ser Gln Val Met Thr His Leu Arg Val Ile Tyr Glu
   450 455 460
   Arg Met Asn Gln Ser Leu Ser Leu Leu Tyr Asn Val Pro Ala Val Ala
   465 470 475 480
   Glu Glu Ile Gln Asp Glu Val Asp Glu Leu Leu Gln Lys Glu Gln Asn
   485 490 495
   Tyr Ser Asp Asp Val Leu Ala Asn Met Ile Ser Glu Pro Arg Ile Ser
   500 505 510
   Tyr Gly Asn Asp Ala Leu Met Pro Ser Leu Thr Glu Thr Lys Thr Thr
   515 520 525
   Val Glu Leu Leu Pro Val Asn Gly Glu Phe Ser Leu Asp Asp Leu Gln
   530 535 540
   Pro Trp His Ser Phe Gly Ala Asp Ser Val Pro Ala Asn Thr Glu Asn
   545 550 555 560
   Glu Val Glu Pro Val Asp Ala Arg Pro Ala Ala Asp Arg Gly Leu Thr
   565 570 575
   Thr Arg Pro Gly Ser Gly Leu Thr Asn Ile Lys Thr Glu Glu Ile Ser
   580 585 590
   Glu Val Lys Met Asp Ala Glu Phe Arg His Asp Ser Gly Tyr Glu Val
   595 600 605
   His His Gln Lys Leu Val Phe Phe Ala Glu Asp Val Gly Ser Asn Lys
   610 615 620
   Gly Ala Ile Ile Gly Leu Met Val Gly Gly Val Val Ile Ala Thr Val
   625 630 635 640
   Ile Val Ile Thr Leu Val Met Leu Lys Lys Lys Gln Tyr Thr Ser Ile
   645 650 655
   His His Gly Val Val Glu Val Asp Ala Ala Val Thr Pro Glu Glu Arg
   660 665 670
   His Leu Ser Lys Met Gln Gln Asn Gly Tyr Glu Asn Pro Thr Tyr Lys
   675 680 685
   Phe Phe Glu Gln Met Gln Asn
   690 695

<210> 2
<211> 534
<212> PRT
<213> Chick

<400> 2
   Gly Met Asn Leu His Asp Tyr Gly Met Leu Leu Pro Cys Gly Ile Asp
   1 5 10 15
   Lys Phe Arg Gly Val Glu Phe Val Cys Cys Pro Leu Ala Glu Glu Ser
   20 25 30
   Asp Asn Leu Asp Ser Ala Asp Ala Glu Asp Asp Asp Ser Asp Val Trp
   35 40 45
   Trp Gly Gly Ala Asp Ala Asp Tyr Ala Asp Gly Ser Asp Asp Lys Val
   50 55 60
   Val Glu Glu Gln Pro Glu Glu Asp Glu Glu Leu Thr Val Val Glu Asp
   65 70 75 80
   Glu Asp Ala Asp Asp Asp Asp Asp Asp Asp Gly Asp Glu Ile Glu Glu
   85 90 95
   Thr Glu Glu Glu Tyr Glu Glu Ala Thr Glu Arg Thr Thr Ser Ile Ala
   100 105 110
   Thr Thr Thr Thr Thr Thr Thr Glu Ser Val Glu Glu Val Val Arg Val
   115 120 125
   Pro Thr Thr Ala Ala Ser Thr Pro Asp Ala Val Asp Lys Tyr Leu Glu
   130 135 140
   Thr Pro Gly Asp Glu Asn Glu His Ala His Phe Gln Lys Ala Lys Glu
   145 150 155 160
   Arg Leu Glu Ala Lys His Arg Glu Arg Met Ser Gln Val Met Arg Glu
                   165                 170                 175
   Trp Glu Glu Ala Glu Arg Gln Ala Lys Asn Leu Pro Lys Ala Asp Lys
               180                 185                 190
   Lys Ala Val Ile Gln His Phe Gln Glu Lys Val Glu Ser Leu Glu Gln
           195                 200                 205
   Glu Ala Ala Asn Glu Arg Gln Gln Leu Val Glu Thr His Met Ala Arg
       210                 215                 220
   Val Glu Ala Met Leu Asn Asp Arg Arg Arg Ile Ala Leu Glu Asn Tyr
   225                 230                 235                 240
   Ile Thr Ala Leu Gln Thr Val Pro Pro Arg Pro Arg His Val Phe Asn
                   245                 250                 255
   Met Leu Lys Lys Tyr Val Arg Ala Glu Gln Lys Asp Arg Gln His Thr
               260                 265                 270
   Leu Lys His Phe Glu His Val Arg Met Val Asp Pro Lys Lys Ala Ala
           275                 280                 285
   Gln Ile Arg Ser Gln Val Met Thr His Leu Arg Val Ile Tyr Glu Arg
       290                 295                 300
   Met Asn Gln Ser Leu Ser Phe Leu Tyr Asn Val Pro Ala Val Ala Glu
   305                 310                 315                 320
   Glu Ile Gln Asp Glu Val Asp Glu Leu Leu Gln Lys Glu Gln Asn Tyr
                   325                 330                 335
   Ser Asp Asp Val Leu Ala Asn Met Ile Ser Glu Pro Arg Ile Ser Tyr
               340                 345                 350
   Gly Asn Asp Ala Leu Met Pro Ser Leu Thr Glu Thr Lys Thr Thr Val
           355                 360                 365
   Glu Leu Leu Pro Val Asp Gly Glu Phe Ser Leu Asp Asp Leu Gln Pro
       370                 375                 380
   Trp His Pro Phe Gly Val Asp Ser Val Pro Ala Asn Thr Glu Asn Glu
   385                 390                 395                 400
   Val Glu Pro Val Asp Ala Arg Pro Ala Ala Asp Arg Gly Leu Thr Thr
                   405                 410                 415
   Arg Pro Gly Ser Gly Leu Thr Asn Val Lys Thr Glu Glu Val Ser Glu
               420                 425                 430
   Val Lys Met Asp Ala Glu Phe Arg His Asp Ser Gly Tyr Glu Val His
           435                 440                 445
   His Gln Lys Leu Val Phe Phe Ala Glu Asp Val Gly Ser Asn Lys Gly
       450                 455                 460
   Ala Ile Ile Gly Leu Met Val Gly Gly Val Val Ile Ala Thr Val Ile
   465                 470                 475                 480
   Val Ile Thr Leu Val Met Leu Lys Lys Lys Gln Tyr Thr Ser Ile His
                   485                 490                 495
   His Gly Val Val Glu Val Asp Ala Ala Val Thr Pro Glu Glu Arg His
               500                 505                 510
   Leu Ser Lys Met Gln Gln Asn Gly Tyr Glu Asn Pro Thr Tyr Lys Phe
           515                 520                 525
   Phe Glu Gln Met Gln Asn
       530

<210> 3
<211> 5
<212> PRT
<213> Artificial Sequence

<220>
<223> Description of Artificial Sequence: 5-mer
      polypeptide

<400> 3
Arg Glu Arg Met Ser
  1               5

<210> 4
<211> 5
<212> PRT
<213> Artificial Sequence

<220>
<223> Description of Artificial Sequence: 5-mer
      polypeptide

<400> 4
Ser Met Arg Glu Arg
  1               5

<210> 5
<211> 5
<212> PRT
<213> Artificial Sequence

<220>
<223> Description of Artificial Sequence: 5-mer
      polypeptide

<400> 5
Arg Ser Ala Glu Arg
  1               5

<210> 6
<211> 17
<212> PRT
<213> Artificial Sequence

<220>
<223> Description of Artificial Sequence: 17-mer
      polypeptide

<400> 6
Ala Lys Glu Arg Leu Glu Ala Lys His Arg Glu Arg Met Ser Gln Val
  1               5                  10                  15

Met

<210> 7

<211> 17
<212> PRT
<213> Artificial Sequence

<220>
<223> Description of Artificial Sequence: 17-mer
      polypeptide

<400> 7
Met Val Gln Ser Met Arg Glu Arg His Lys Ala Glu Leu Arg Glu Lys
1               5                   10                  15

Ala

<210> 8
<211> 17
<212> PRT
<213> Artificial Sequence

<220>
<223> Description of Artificial Sequence: 17-mer
      polypeptide

<400> 8
Val His His Gln Lys Leu Val Phe Phe Ala Glu Asp Val Gly Ser Asn
1               5                   10                  15

Lys

<210> 9
<211> 3
<212> PRT
<213> Artificial Sequence

<220>
<223> Description of Artificial Sequence: 3-mer polypeptide

<400> 9
Arg Glu Arg
1

<210> 10
<211> 4
<212> PRT
<213> Artificial Sequence

<220>
<223> Description of Artificial Sequence: 4-mer polypeptide

<400> 10
Arg Glu Arg Met
1

<210> 11
<211> 4
<212> PRT
<213> Artificial Sequence

<220>
<223> Description of Artificial Sequence: 4-mer polypeptide

<400> 11
Met Arg Glu Arg
1

<210> 12
<211> 16
<212> PRT
<213> Artificial Sequence

<220>
<223> Description of Artificial Sequence: 16-mer
      polypeptide

<400> 12
Gln Lys Ala Lys Glu Arg Leu Glu Ala Lys His Arg Glu Arg Met Ser
1               5                   10                  15

<210> 13
<211> 15
<212> PRT
<213> Artificial Sequence

<220>
<223> Description of Artificial Sequence: 15-mer
      polypeptide

<400> 13
Glu Arg Leu Glu Ala Lys His Arg Glu Arg Met Ser Gln Val Met
1               5                   10                  15

<210> 14
<211> 14
<212> PRT
<213> Artificial Sequence

<220>
<223> Description of Artificial Sequence: 14-mer
      polypeptide

<400> 14
Ala Lys Glu Arg Leu Glu Ala Lys His Arg Glu Arg Met Ser
1               5                   10

<210> 15
<211> 12
<212> PRT
<213> Artificial Sequence

<220>
<223> Description of Artificial Sequence: 12-mer
      polypeptide

<400> 15
Glu Arg Leu Glu Ala Lys His Arg Glu Arg Met Ser
1               5                   10

<210> 16
<211> 11

<212> PRT
<213> Artificial Sequence

<220>
<223> Description of Artificial Sequence: 11-mer
      polypeptide

<400> 16
Glu Arg Leu Glu Ala Lys His Arg Glu Arg Met
  1               5                   10



<210> 17
<211> 11
<212> PRT
<213> Artificial Sequence

<220>
<223> Description of Artificial Sequence: 11-mer
      polypeptide

<400> 16
Ala Lys His Arg Glu Arg Met Ser Gln Val Met
  1               5                   10



<210> 18
<211> 10
<212> PRT
<213> Artificial Sequence

<220>
<223> Description of Artificial Sequence: 10-mer
      polypeptide

<400> 18
Glu Arg Leu Glu Ala Lys His Arg Glu Arg
  1               5                   10



<210> 19
<211> 6
<212> PRT
<213> Artificial Sequence

<220>
<223> Description of Artificial Sequence: 6-mer
      polypeptide

<400> 19
His Arg Glu Arg Met Ser
  1               5



<210> 20
<211> 22
<212> PRT
<213> Artificial Sequence

<220>
<223> Description of Artificial Sequence: 22-mer
      polypeptide

<400> 20
Phe Gln Lys Ala Lys Glu Arg Leu Glu Ala Lys His Arg Glu Arg Met

1                   5                   10                  15

Ser Gln Val Met Arg Glu
                20

<210> 21
<211> 16
<212> DNA
<213> Artificial Sequence

<220>
<223> Description of Artificial Sequence: end-protected
      16-mer oligodeoxynucleotide modified at positions
      2 & 13 to prevent internal looping

<400> 21
cccgaggact gagcca                                                        16

<210> 22
<211> 40
<212> PRT
<213> Artificial Sequence

<220>
<223> Description of Artificial Sequence: 40-mer
      polypeptide

<400> 22
Thr Pro Asp Ala Val Asp Lys Tyr Leu Glu Thr Pro Gly Asp Glu Asn
  1               5                  10                  15

Glu His Ala His Phe Gln Lys Ala Lys Glu Arg Leu Glu Ala Lys His
             20                  25                  30

Arg Glu Arg Met Ser Gln Val Met
         35                  40

## Description

### FIELD OF THE INVENTION

The present invention relates to polypeptide compounds, to compounds derived therefrom and to the use of such compounds in medicine. Compounds according to the invention are believed to be potentially useful as cognitive enhancers and in the treatment of neurodegenerative diseases such as Alzheimer's disease.

### BACKGROUND TO THE INVENTION

Alzheimer's disease is a degenerative brain disease which is characterised by progressive loss of memory and subsequently most other cognitive functions in an irreversible decline over a period of years. It represents a substantial health problem, particularly in an ageing population.

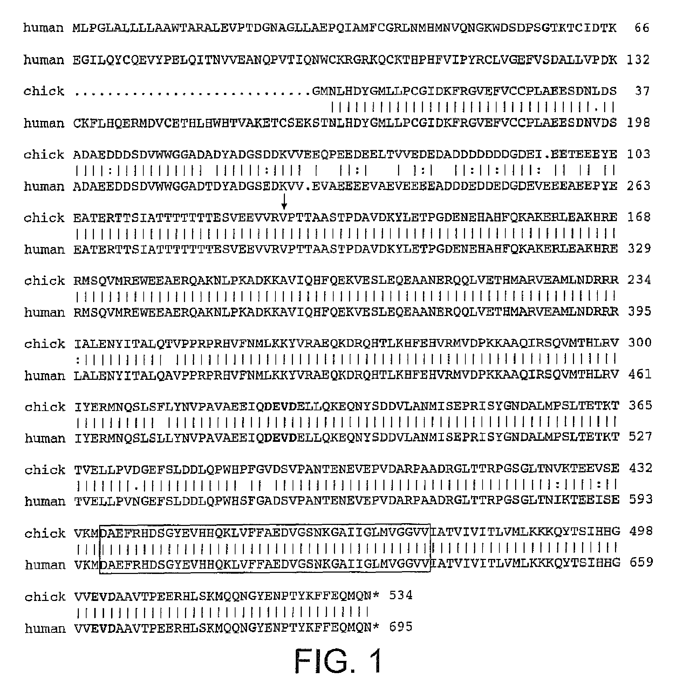

The amyloid precursor protein ("APP") is a multifunctional transmembrane protein and is known to have important functions in normal brain tissue. The human form of APP is known to consist of 695 amino acid residues (SEQ ID No: 1) in a sequence which is also known (see Kang et al, Nature 325, 733-736 (1987), the contents of which are incorporated herein by reference). The chick form of APP is known to consist of 534 amino acid residues (SEQ ID No: 2) and to resemble the human form closely, being approximately 95% homologous therewith (see the paper by Kang et al just mentioned and Barnes et al, J Neurosci, 18 (15) 5869-5880 (1998), contents of which are also incorporated herein by reference). The amino acid sequences of the human and chick forms of APP are reproduced in figure 1 of the drawings of this specification.

Two effects which have been noted to take place in the brain of a person suffering from Alzheimer's disease are the build up outside the nerve cells of the brain of tangled masses of protein and the build up inside the brain cells of a different protein. The extracellular proteins are known to be aggregates of polypeptides having amino acid sequences corresponding to portions of the extracellular part of APP. The tangled masses of these proteins are known as amyloid plaques. The intracellular proteins are known as tau proteins. It is however not known whether either or both of the extracellular accumulation of amyloid plaques and the intracellular accumulation of tau proteins are the causes or the symptoms of Alzheimer's and related neurodegenerative diseases of the Alzheimer type.

The amino acid sequence of the β-amyloid polypeptide fragment (1-42) is identical in the human and chick forms of APP and consists of amino acid residues 597 to 638 in the human form and residues 436 to 477 in the chick form, (see the papers by Kang et al and Barnes et al referred to hereinbefore).

### DEFINITIONS

The following expressions are used in this specification and have the following meanings, except where the context indicates otherwise:

| | |
|---|---|
| **APP** | means "amyloid precursor protein"; |
| **human APP** | means the human form of APP; |
| **chick APP** | means the chick form of APP; |
| **RERMS** | means the pentapeptide Arg-Glu-Arg-Met-Ser (SEQ ID No: 3); |
| **APP 328-332** | also means the pentapeptide Arg-Glu-Arg-Met-Ser (SEQ ID No: 3) which corresponds to amino acid residues 328 to 332 of human APP; |
| **SMRER** | means the pentapeptide Ser-Met-Arg-Glu-Arg (SEQ ID No: 4); |
| **APP 332-328** | means the same as SMRER; |
| **A**β **domain** | means the domain of APP which forms β-amyloid plaques; |
| β**-amyloid 12-28** | means the sequence of amino acid residues which constitute part of the Aβ domain of human APP, the sequence being Val-His-His-Gln-Lys-Leu-Val-Phe-Phe-Ala-Glu-Asp-Val-Gly-Ser-Asn-Lys( SEQ ID No: 8) which corresponds to amino acid residues 608 to 624 of human APP and amino acids 447 to 463 of chick APP; |
| **RSAER** | means the pentapeptide Arg-Ser-Ala-Glu-Arg (SEQ ID No: 5); |
| **APP 319-335** | means the polypeptide Ala-Lys-Glu-Arg-Leu-Glu-Ala-Lys-His-Arg-Glu-Arg-Met-Ser-Gln-Val-Met (AKERLEAKHRERMSQVM)(SEQ ID No: 6); |
| **RER** | means the tripeptide Arg-Glu-Arg (SEQ ID No: 9); |
| **APP 328-330** | means the same as RER; |
| **Ac-RER** | means RER in which one of the hydrogen atoms of the -NH₂ group at the N-terminus has been replaced by an acetyl group; the structural formula of Ac-RER is given hereinbelow; |
| **RERM** | means the tetrapeptide Arg-Glu- |
| **APP 328-331** | Arg-Met (SEQ ID No: 10); means the same as RERM; |
| **MRER** | means the tetrapeptide Met-Arg-Glu-Arg (SEQ ID No: 11); |
| **APP 331-328** | means the same as MRER; |
| **amino acid** | as used herein is meant to include both natural and synthetic amino acids, and both D and L amino acids; |
| **standard amino acid** | means any of the twenty standard L-amino acids commonly found in naturally occurring peptides; |
| **nonstandard amino acid** | means any amino acid, other than the standard amino acids, regardless of whether it is prepared synthetically or derived from a natural source; as used herein, "synthetic amino acid" also encompasses chemically modified amino acids, including but not limited to salts, amino acid derivatives (such as amides), and substitutions. Amino acids contained within the peptides of the present invention, and particularly at the carboxy- or amino-terminus, can be modified by methylation, amidation, acetylation or substitution with other chemical groups which can change the peptide's circulating half life without adversely affecting their activity; additionally, a disulfide linkage may be present or absent in the peptides of the invention; |
| **derivative** | includes any purposefully generated peptide which in its entirety, or in part, has a substantially similar amino acid sequence to the present compounds; derivatives of the present compounds may be characterized by single or multiple amino acid substitutions, deletions, additions, or replacements; these derivatives may include (a) derivatives in which one or more amino acid residues of the present compounds are substituted with conservative or non-conservative amino acids; (b) derivatives in which one or more amino acids are added to the present compounds; (c) derivatives in which one or more of the amino acids of the present compounds include a substituent group; (d) derivatives in which the present compounds or a portion thereof is fused to another peptide (e.g., serum albumin or protein transduction domain); (e) derivatives in which one or more nonstandard amino acid residues (*i*.*e.*, those other than the 20 standard L-amino acids found in naturally occurring proteins) are incorporated or substituted into the present compounds sequence; and (f) derivatives in which one or more nonamino acid linking groups are incorporated into or replace a portion of the present compounds; |
| **APP 296-335,APP 317-332, APP 321-335, APP 319-332, APP 321-332, APP 321-331, APP 325-335, APP 321-330, APP 327-332 and APP 316-337** | have the meanings given in tables 1 and 2 hereinafter. |

Throughout this specification and its claims amino acid sequences are written using the standard one-letter or three-letter abbreviations. All sequences are written from left to right in the direction from the N-terminus to the C-terminus.

The following term is defined as follows:

| | |
|---|---|
| **reverse order sequence** | as used herein, the reverse order sequence of a given sequence is a sequence in which the order of amino acid residues is reversed compared with the given sequence when reading in the direction from the N-terminus to the C-terminus and *vice versa;* thus, for example, SMRER is the reverse order sequence of RERMS, each being read as stated above from left to right |
| | in the N-terminal to C-terminal direction; further, MVQSMRERHKAELREKA (SEQ ID No: 7) (also referred to herein as APP 335-319) is the reverse order sequence of APP 319-335 defined above. |

WO-A-94/09808 (The Regents of The University of California, inventor T Saito) and T Saito and various coauthors in J. Neuroscience 14 5461-5470 (1994), J. Neuobiol. 25, 585-594 (1994) and J. Cell Biol. 121, 879-886 (1993) disclose certain polypeptides corresponding to parts of human APP. These polypeptides consist of the following:

**Table 1**

| no. of amino acid residues | corresponding part of human APP | sequence | SEQ ID NO. |
|---|---|---|---|
| 40 | APP 296-335 | TPDAVDKYLETPGDENEHAHF QKAKERLEAKHRERMSQVM [Thr-Pro-Asp-Ala-Val- Asp-Lys-Tyr-Leu-Glu- Thr-Pro-Gly-Asp-Glu- Asn-Glu-His-Ala-His- Phe-Gln-Lys-Ala-Lys- Glu-Arg-Leu-Glu-Ala- Lys-His-Arg-Glu-Arg- Met-Ser-Gln-Val-Met] | 22 |
| 17 | APP 319-335 | AKERLEAKHRERMSQVM [Ala-Lys-Glu-Arg-Leu- Glu-Ala-Lys-His-Arg- Glu-Arg-Met-Ser-Gln- Val-Met] | 6 |
| 16 | APP 317-332 | QKAKERLEAKHRERMS [Gln-Lys-Ala-Lys-Glu- Arg-Leu-Glu-Ala-Lys- His-Arg-Glu-Arg-Met- Ser] | 12 |
| 15 | APP 321-335 | ERLEAKHRERMSQVM [Glu-Arg-Leu-Glu-Ala- Lys-His-Arg-Glu-Arg- Met-Ser-Gln-Val-Met] | 13 |
| 14 | APP 319-332 | AKERLEAKHRERMS [Ala-Lys-Glu-Arg-Leu- Glu-Ala-Lys-His-Arg- Glu-Arg-Met-Ser] | 14 |
| 12 | APP 321-332 | ERLEAKHRERMS [Glu-Arg-Leu-Glu-Ala- Lys-His-Arg-Glu-Arg- Met-Ser] | 15 |
| 11 | APP 321-331 | ERLEAKHRERM [Glu-Arg-Leu-Glu-Ala- Lys-His-Arg-Glu-Arg- Met] | 16 |
| 11 | APP 325-335 | AKHRERMSQVM [Ala-Lys-His-Arg-Glu- Arg-Met-Ser-Gln-Val- Met] | 17 |
| 10 | APP 321-330 | ERLEAKHRER [Glu-Arg-Leu-Glu-Ala- Lys-His-Arg-Glu-Arg] | 18 |
| 6 | APP 327-332 | HRERMS [His-Arg-Glu-Arg-Met- Ser] | 19 |
| 5 | APP 328-332 | RERMS [Arg-Glu-Arg-Met-Ser] | 3 |
| 4 | APP 328-331 | RERM [Arg-Glu-Arg-Met] | 10 |
| 3 | APP 328-330 | RER [Arg-Glu-Arg] | 9 |

The same publications also refer to a 17-mer polypeptide which is the reverse-order sequence of APP 319-335 (as defined above). The reverse order sequence is also identified herein as APP 335-319.

G Multhorp et al is J. Mol Recognition 8, 247-257 (1995) disclose the following polypeptide which also corresponds to part of human APP:

**Table 2**

| No. of amino acid residues | corresponding part of human APP | Sequence | SEQ ID NO. |
|---|---|---|---|
| 22 | APP 316-337 | FQKAKERLEAKHRERMSQVMRE [Phe-Gln-Lys-Ala-Lys- Glu-Arg-Leu-Glu-Ala- Lys-His-Arg-Glu-Arg- Met-Ser-Gln-Val-Met- Arg-Glu] | 20 |

### SUMMARY OF THE INVENTION

The present invention provides a derivative of a polypeptide having the formula:

X₁-Arg-Xaa-Arg-X₂ (I)

wherein X₁ and X₂, which may be the same or different, each represents from zero to 30 natural or synthetic amino acid residues or derivatives thereof and Xaa represents a natural or synthetic amino acid residue or a derivative thereof, at least one functional group of at least one said amino acid residue or derivative thereof being protected by a protective group.

Preferably, the protected functional group(s) comprise one or more amino groups.

Advantageously, the amino group(s) comprise an N-terminal amino group.

Conveniently, at least one protected amino group is protected by replacement of only one of its hydrogen atoms by a protective group.

In other polypeptide derivatives, the protected functional group(s) comprise one or more carboxyl groups.

Advantageously, the carboxyl group(s) comprise a C-terminal carboxyl group.

Advantageously, the or each protective group is an acyl group represented by the formula 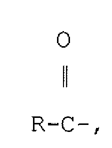 wherein R represents a straight- or branched-chain alkyl group, for example a methyl, ethyl, n-propyl, isopropyl, n-butyl, isobutyl, s-butyl, t-butyl, pentyl or hexyl group, a substituted or unsubstituted cycloalkyl group, for example a methylcyclohexyl or cyclohexyl group, a substituted or unsubstituted straight- or branched-chain aralkyl group, for example a benzyl group, or a substituted or unsubstituted aryl group, for example a phenyl or tolyl group. Examples of substituents in the substituted groups mentioned above are the alkyl groups also mentioned above.

Straight- or branched-chain alkyl groups are most preferred for R, methyl groups being particularly preferred.

In formula (I), amino acid derivatives include, for example, substituted amino acids.

In formula (I), X₁ and X₂ are each preferably idenpendently from zero to 20, more preferably from zero to 10.

When X₁ and X₂ are both zero in formula (I), formula (I) is that of a tripeptide derivative which is preferably Ac-RER when Xaa is glutamic acid.

Preferably, compounds according to the invention are compounds in which X₁ and X₂ are such that formula (I) represents an amino acid sequence which is identical or closely homologous to amino acid residues 328 to 332 of human APP and up to 30 successive amino acid residues of human APP extending in each direction therefrom, or to a sequence which is closely homologous thereto or in which the amino acids thereof are replaced by nonstandard amino acids.

It is also preferred that formula (I) is one in which X₁ and X₂ are such that the formula represents a reverse-order amino acid sequence which is identical or closely homologous to amino acid residues 330 to 328 of human APP and up to 30 successive amino acid residues of human APP extending in each direction therefrom, or to a sequence which is closely homologous thereto or in which the amino acids thereof are replaced by nonstandard amino acids.

As used herein, a peptide or a portion of a peptide which is "closely homologous" means the peptide, or the portion thereof, has an amino acid homology of greater than about 80% with respect to a reference peptide, preferably greater than about 90% and, more preferably, greater than about 95%.

Amino acid sequence homology may be computed by using the BLASTP and TBLASTN programs which employ the BLAST (basic local alignment search tool) 2.0.14 algorithm; BLASTP and TBLASTN settings to be used in such computations are indicated in table 3 below. Amino acid sequence identity (complete homology) is reported under "Identities" by the BLASTP and TBLASTN programs. Amino acid sequence similarity (degree of homology) is reported under "Positives" by the BLASTP and TBLASTN programs. Techniques for computing amino acid sequence homology are well known to those skilled in the art, and the use of the BLAST algorithm is described in Altschul et al. (1990), J. Mol. Biol. 215: 403-10 and Altschul et al. (1997), Nucleic Acids Res. 25:3389-3402, the disclosures of which are herein incorporated by reference in their entirety. BLASTP and TBLASTN programs utilizing the BLAST 2.0.14 algorithm and may be accessed at http://www.ncbi.nlm.nih.gov/.

**Table 3 - Settings to be used for the computation of amino acid sequence similarity or identity with BLASTP and TBLASTN programs utilizing the BLAST 2.0.14 algorithm**

| | |
|---|---|
| Expect Value | 10 |
| Filter | Low complexity filtering using SEG program* |
| Substitution Matrix | BLOSUM62 |
| Gap existence cost | 11 |
| Per residue gap cost | 1 |
| Lambda ratio | 0.85 |
| Word size | 3 |

| | |
|---|---|
| *The SEG program is described by Wootton and Federhen (1993), Comput. Chem. 17: 149-163. | |

Preferably, X₁ in formula (I) represents:

X₃-Ala-Lys-Glu-Arg-Leu-Glu-Ala-Lys-His

wherein X₃ represents from zero to 21 natural or synthetic amino acid residues or derivatives thereof, and/or X₂ represents

Met-Ser-Gln-Val-Met-X₄

wherein X₄ represents from zero to 25, natural or synthetic amino acid residues or derivatives thereof.

X₃ and X₄ are again preferably each independently from zero to 20, more preferably from zero to 10.

When X₃ and X₄ are both zero and Xaa is glutamic acid, the amino acid residues of the formula corresponds to the sequence of amino acid residues 319 to 335 of human APP.

It is also preferred that X₁ in (I) represents:

X₃-Met-Val-Gln-Ser-Met

wherein X₃ represents from zero to 25, natural or synthetic amino acid residues or derivatives thereof, and/or X₂ represents:

His-lys-Ala-Glu-Leu-Arg-Glu-Lys-Ala-X₄

wherein X₄ represents from zero to 21, natural or synthetic amino acid residues or derivatives thereof.

X₃ and X₄ are again preferably idependently each from zero to 20, more preferably from zero to 10.

When X₃ and X₄ are both zero and Xaa is glutamic acid, the amino acid residues of the formula corresponds to the reverse-order sequence of amino acid residues 335 to 319 of human APP.

The invention also provides compounds of formula (I) in which X₂ represents:

Met - X₄

wherein X₄ represents from zero to 29 natural or synthetic amino acid residues or derivatives thereof.

In such compounds, X₂ preferably represents:

Met-Ser-X₄

wherein X₄ represents from zero to 28 natural or synthetic amino acid residues or derivatives thereof.

Further, the invention provides compounds of formula (I) in which X₁ represents:

X₃-Met

wherein X₃ represents from zero to 29 natural or synthetic amino acid residues or derivatives thereof.

In such compounds, X₁ preferably represents:

X₃-Ser-Met

wherein X₃ represents from zero to 28 natural or synthetic amino acid residues or derivatives thereof.

The invention thus provides derivatives of the stated kind of the peptides RER, RERM, MRER, RERMS and SMRER.

The most preferred of such derivatives is the compound Ac-RER which has the structural formula:

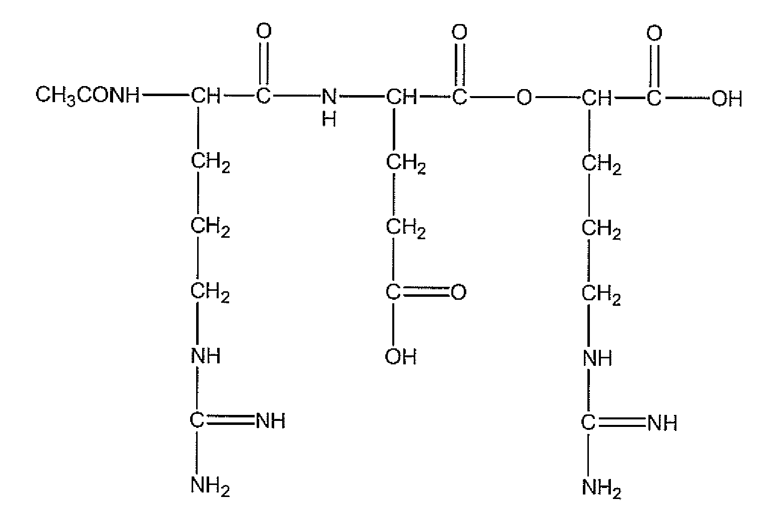

In addition to the compounds mentioned hereinbefore, the present invention also provides the compounds (including the stated derivatives of RER, RERM, MRER, SMRER and RERMS) for use in medicine and their use in the preparation of medicaments for the treatment of neurodegenerative diseases, including Alzheimer's disease, and as cognitive enhancers.

The invention further provides pharmaceutical compositions comprising the compounds (including the stated derivatives of RER, RERM, MRER, SMRER and RERMS) and a pharmaceutically-acceptable carrier and also compositions
in which a compound according to the invention (including the stated derivatives of RER, RERM, MRER, SMRER and RERMS) is chemically or physically linked to a further molecule or vehicle to form a complex for pharmaceutical delivery of the compound.

Further, the invention provides a method of treatment of a neurodegenerative disease in a human or non-human animal suffering or potentially suffering from the disease is administered with an amount of a composition referred to in the preceding paragraph or a compound according to the present invention effective to treat the disease. The invention also provides a method of producing a cognitive enhancement in a human or non-human animal by administering to the animal a said composition or compound in an amount effective to produce the enhancement.

The compounds which are most preferred in the medical uses and pharmaceutical compositions are the stated derivatives of the following:

Arg-Glu-Arg (SEQ ID No: 9)

which corresponds to amino acid residues 328-330 of human APP,

Arg-Glu-Arg-Met (SEQ ID No: 10)

which corresponds to amino acid residues 328-331 of human APP,

Met-Arg-Glu-Arg (SEQ ID No: 11)

which is the reverse-order polypeptide corresponding to the above,

Arg-Glu-Arg-Met-Ser (SEQ ID No: 3)

which corresponds to amino acid residues 328-332 of human APP,

Ser-Met-Arg-Glu-Arg (SEQ ID No: 4)

which is the reverse-order polypeptide corresponding to the above, 
Ala-Lys-Glu-Arg-Leu-Glu-Ala-Lys-His-Arg-Glu-Arg-
Met-Ser-Gln-Val-Met                    (SEQ ID No: 6)
 which corresponds to amino acid residues 319-335 of human APP, and 
Met-Val-Gln-Ser-Met-Arg-Glu-Arg-His-Lys-Ala-Glu-
Leu-Arg-Glu-Lys-Ala (SEQ ID No: 7)
 which is the reverse-order polypeptide corresponding to the above.

The present invention also provides a compound having a formula comprising:

X₁-Arg-Glu-Arg-X₂ (II)

wherein X₁ and X₂, which may be the same or different, each represents from zero to 32 natural or synthetic amino acid residues or derivatives thereof, and Xaa represents a natural or synthetic amino acid residue or a derivative thereof, it being preferred that the compound is none of the following (as hereinbefore defined): APP 296-335, APP 319-335, APP 317-332, APP 321-335, APP 319-332, APP 321-332, APP 321-331, APP 325-335, APP 321-330, APP 327-332, APP 328-332, APP 328-331, APP 328-330, APP 335-319 and APP 316-337.

In formula (II), X₁ and X₂ are preferably and idependently each from zero to 22, more preferably from zero to 12. Most preferably, one or both of X₁ and X₂ is zero.

Preferably, X₁ and X₂ are such that formula (II) represents an amino acid sequence which is identical or closely homologous to amino acid residues 328 to 330 of human APP and up to 32 successive amino acid residues of human APP extending in each direction therefrom, the formula also comprising sequences closely homologous to said sequence and sequences in which said amino acids thereof are replaced by nonstandard amino acids and/or by derivatives of acid amino acids.

It is also preferred that X₁ and X₂ are such that formula (II) represents a reverse-order amino acid sequence which is identical or closely homologous to amino acid residues 330 to 328 of human APP and up to 32 successive amino acid residues of human APP extending in each direction therefrom, the formula also comprising sequences closely homologous to said reverse-order sequence and sequences in which said amino acids thereof are replaced by nonstandard amino acids and/or by derivatives of acid amino acids.

The invention further provides a compound having a formula comprising:

X₁-Arg-Xaa-Arg-X₂ (III)

wherein X₁ and X₂, which may be the same or different, each represents from zero to 32 natural or synthetic amino acid residues or derivatives thereof and Xaa represents a natural or synthetic amino acid residue or a derivative thereof, for use in medicine.

In formula (III), X₁ and X₂ are preferably idependently each from zero to 22, more preferably from zero to 12.
Most preferably, one or both of X₁ and X₂ is zero.

Preferably, X₁ and X₂ are such that formula (III) represents an amino acid sequence which is identical or closely homologous to amino acid residues 328 to 330 of human APP and up to 32 successive amino acid residues of human APP extending in each direction therefrom, the formula also comprising sequences closely homologous to said sequence and sequences in which said amino acids thereof are replaced by nonstandard amino acids and/or by derivatives of acid amino acids.

It is also preferred that X₁ and X₂ are such that formula (III) represents a reverse-order amino acid sequence which is identical or closely homologous to amino acid residues 330 to 328 of human APP and up to 30 successive amino acid residues of human APP extending in each direction therefrom, the formula also comprising sequences closely homologous to said reverse-order sequence and sequences in which said amino acids thereof are replaced by nonstandard amino acids and/or by derivatives of acid amino acids.

A particularly preferred compound is one in which X₁ and X₂ represents:

X₃-Ala-Lys-Glu-Arg-Leu-Glu-Ala-Lys-His

wherein X₃ represents from zero to 23 natural or synthetic amino acid residues or derivatives thereof, and/or X₂ represents:

Met-Ser-Gln-Val-Met-X₄

wherein X₄ represents from zero to 27 natural or synthetic amino acid residues or derivatives thereof.

Another preferred compound is one in which X₁ and X₂ represents:

X₃-Met-Val-Gln-Ser-Met

wherein X₃ represents from zero to 27 natural or synthetic amino acid residues or derivatives thereof, and/or X₂ represents:

His-Lys-Ala-Glu-Leu-Arg-Glu-Lys-Ala-X₄

wherein X₄ represents from zero to 23 natural or synthetic amino acid residues or derivatives thereof.

In such compounds, X₃ and X₄ are preferably idependently each from zero to 20, more preferably from zero to 10. Most preferably, one or both of X₃ and X₄ is zero.

Xaa in formulae (I) and (III) is preferably glutamic acid.

The present invention also provides the use of a compound having a formula comprising:

X₁-Arg-Xaa-Arg-X₂ (III)

wherein X₁ and X₂, which may be the same or different, each represents from zero to 32 natural or synthetic amino acid residues or derivatives thereof and Xaa represents a natural or synthetic amino acid residue or a derivative thereof, in the preparation of a medicament for use in the treatment of a neurodegenerative disease or as a cognitive enhancer.

In addition, the invention provides a pharmaceutical composition comprising such a compound and a pharmaceutically acceptable carrier.

Moreover, the invention provides a said compound which is chemically or physically linked to a further molecule or vehicle to form a complex for pharmaceutical delivery of the compound.

Furthermore, the invention provides methods of treating a neurodegenerative disease or of obtaining a cognitive enhancement in a human or non-human animal by administering to the animal an effective amount of a compound according to the invention, optionally in the form of a pharmaceutical composition as referred to.

The present invention provides a compound having a formula comprising:

X₁ Arg-Xaa₁-Arg-Xaa₂-Xaa₃-X₂ (IV)

wherein X₁ and X₂, which may be the same or different, each represents from zero to 30 natural or synthetic amino acid residues or derivatives thereof and Xaa₁, Xaa₂ and Xaa₃, which may be the same or different, each represents a natural or synthetic amino acid or a derivative thereof.

The amino acid derivatives include, for example, substituted amino acids.

X₁ and X₂ are each preferably from zero to 20, more preferably from zero to 10.

When X₁ and X₂ are both zero, the formula is that of a pentapeptide which is RERMS when Xaa₁ is glutamic acid, xaa₂ is methionine and Xaa₃ is serine.

In a compound according to the invention, Xaa₁ is preferably glutamic acid, Xaa₂ is preferably methionine and Xaa₃ is preferably serine.

Preferably, the compound is one in which X₁ and X₂ are such that (IV) represents an amino acid sequence which is identical or closely homologous to amino acid residues 328 to 332 of human APP and up to 30 successive amino acid residues of human APP extending in each direction therefrom.

The degree of homology is preferably at least 80%, more preferably at least 90% and, most preferably, at least 95%.

The invention also provides compounds having a formula comprising:

X₁-Xaa₃-Xaa₂-Arg-Xaa₁-Arg-X₂ (V)

wherein X₁, X₂, Xaa₁, Xaa₂ and Xaa₃ are as stated hereinbefore.

When X₁ and X₂ are both zero, the formula is that of a pentapeptide which is SMRER when Xaa₁ is glutamic acid, Xaa₂ is methionine and Xaa₃ is serine.

Such formulae represent the reverse-order sequences of the formulae mentioned hereinbefore.

Preferably, the compound is one in which X₁ and X₂ are such that the formula represents a reverse-order amino acid sequence which is identical or closely homologous to amino acid residues 332 to 328 of human APP and up to 30 successive amino acid residues of human APP extending in each direction therefrom.

The degree of homology is again preferably at least 80%, more preferably at least 90% and, most preferably, at least 95%.

Preferably, X₁ in (IV) represents:

X₃-Ala-Lys-Glu-Arg-Leu-Glu-Ala-Lys-His

and/or X₂ represents

Gln-Val-Met-X₄

X₃ and X₄ being the same or different and representing from zero to 30 natural or synthetic amino acid residues or derivatives thereof.

X₃ and X₄ are again preferably each from zero to 20, more preferably from zero to 10.

When X₃ and X₄ are both zero and Xaa₁, Xaa₂ and Xaa₃ are glutamic acid, methionine and serine, respectively, the formula corresponds to the sequence of amino acid residues 319 to 335 of human APP.

Preferably, X₂ in (V) represents:

X₃-Met-Val-Gln

and/or X₂ represents:

His-Lys-Ala-Glu-Leu-Arg-Glu-Lys-Ala-X₄

wherein X₃ and X₄, which may be the same or different, each represents from zero to 30 natural or synthetic amino acid residues or derivatives thereof.

X₃ and X₄ are again preferably each from zero to 20, more preferably from zero to 10.

When X₃ and X₄ are both zero and Xaa₁, Xaa₂ and Xaa₃ are glutamic acid, methionine and serine, respectively, the formula corresponds to the reverse-order sequence of amino acid residues 335 to 319 of human APP.

In addition to the compounds mentioned hereinbefore, the present invention also provides the compounds (including RERMS and SMRER) for use in medicine and their use in the preparation of medicaments for the treatment of neurodegenerative diseases, including Alzheimer's disease, and as cognitive enhancers.

The invention further provides pharmaceutical compositions comprising the compounds (including RERMS and SMRER) and a pharmaceutically-acceptable carrier and also compositions in which a compound according to the invention (including RERMS and SMRER) is chemically or physically linked to a further molecule or vehicle to form a complex for pharmaceutical delivery of the compound.

The compounds which are most preferred in the medical uses and pharmaceutical compositions are the following:

Arg-Glu-Arg-Met-Ser (SEQ ID No:3)

which corresponds to amino acid residues 328-332 of human APP,

Ser-Met-Arg-Glu-Arg (SEQ ID No:4)

which is the reverse-order polypeptide of the above, 
Ala-Lys-Glu-Arg-Leu-Glu-Ala-Lys-His-Arg-Glu-Arg-Met-Ser-
Gln-Val-Met (SEQ ID No:6)
 which corresponds to amino acid residues 319-335 of human APP, and 
Met-Val-Gln-Ser-Met-Arg-Glu-Arg-His-Lys-Ala-Glu-Leu-Arg-
Glu-Lys-Ala (SEQ ID No:7)
 which is the reverse-order polypeptide of the above.

The present invention provides a compound having a formula comprising:

X₁-Arg-Xaa-Arg-X₂ (VI)

wherein X₁ and X₂, which may be the same or different, each represents from zero to 30 natural or synthetic amino acid residues or derivatives thereof and Xaa represents a natural or synthetic amino acid or a derivative thereof. Xaa is preferably glutamic acid.

The amino acid derivatives include, for example, substituted amino acids.

X₁ and X₂ are each preferably from zero to 20, more preferably from zero to 10.

When X₁ and X₂ are both zero, the formula is that of a tripeptide which is Arg-Glu-Arg (RER) when Xaa is glutamic acid.

Preferably, the compound is one in which X₁ and X₂ are such that (I) represents an amino acid sequence which is identical or closely homologous to amino acid residues 328 to 330 of human APP and up to 30 successive amino acid residues of human APP extending in each direction therefrom.

It is also preferred that the compound is one in which X₁ and X₂ are such that the formula represents a reverse-order amino acid sequence which is identical or closely homologous to amino acid residues 330 to 328 of human APP and up to 30 successive amino acid residues of human APP extending in each direction therefrom.

In each case, the degree of homology is again preferably at least 80%, more preferably at least 90% and, most preferably, at least 95%.

Preferably, X₁ in (VI) represents:

X₃-Ala-Lys-Glu-Arg-Leu-Glu-Ala-Lys-His

and/or X₂ represents

Met-Ser-Gln-Val-Met-X₄

X₃ and X₄ being the same or different and representing from zero to 30 natural or synthetic amino acid residues or derivatives thereof.

X₃ and X₄ are again preferably each from zero to 20, more preferably from zero to 10.

When X₃ and X₄ are both zero and Xaa is glutamic acid, the formula corresponds to the sequence of amino acid residues 319 to 335 of human APP.

It is also preferred that X₁ in (VI) represents:

X₃-Met-Val-Gln-Ser-Met

and/or X₂ represents:

His-Lys-Ala-Glu-Leu-Arg-Glu-Lys-Ala-X₄

wherein X₃ and X₄, which may be the same or different, each represents from zero to 30 natural or synthetic amino acid residues or derivatives thereof.

X₃ and X₄ are again preferably each from zero to 20, more preferably from zero to 10.

When X₃ and X₄ are both zero and Xaa is glutamic acid, the formula corresponds to the reverse-order sequence of amino acid residues 335 to 319 of human APP.

The invention also provides compounds having the formula (VI) in which Xaa is glutamic acid and either X₁ is methionine and X₂ is zero, or X₁ is zero and X₂ is methionine. These are the compounds Met-Arg-Glu-Arg (MRER) (SEQ ID No: 11) and Arg-Glu-Arg-Met (RERM) (SEQ ID No: 10), respectively.

The present invention also provides the compounds mentioned above (including RER) for use in medicine and their use in the preparation of medicaments for the treatment of neurodegenerative diseases, including Alzheimer's disease, and as cognitive enhancers.

The invention further provides pharmaceutical compositions comprising the compounds (including RER) and a pharmaceutically-acceptable carrier and also compositions in which a compound according to the invention (including RER) is chemically or physically linked to a further molecule or vehicle to form a complex for pharmaceutical delivery of the compound.

The compound which is most preferred in the medical uses and pharmaceutical compositions is:

Arg-Glu-Arg (SEQ ID No:9)

which corresponds to amino acid residues 328-330 of human APP.

The invention further provides a compound having a formula comprising

X₁-Ser-Met-Arg-Glu-Arg-X₂ (VII)

wherein X₁ and X₂, which may be the same or different, each represents from zero to 30 amino acid residues, the amino acid residues of X₁ and X₂ being such that, when X₁ and X₂ are not both zero, the formula represents a reverse-order sequence corresponding to amino acid residues 332 to 328 of human APP and from zero to 30 successive amino acid residues of human APP extending in each direction therefrom, the formula also comprising sequences closely homologous to said reverse-order sequence and sequences in which said amino acids thereof are replaced by nonstandard amino acids and/or by derivatives of said amino acids, provided always that the compound is not

Met-Val-Gln-Ser-Met-Arg-Glu-Arg-His-Lys-Ala-Glu-
Leu-Arg-Glu-Lys-Ala (SEQ ID No: 7)

Subject to the above proviso, formula (VII) thus includes within its scope polypeptides which consist of a core sequence of the five amino acid residues 332 to 328 of human APP in reverse order relative to human APP and, extending therefrom in the N-terminal direction, up to 30 of amino acid residues 333 to 362 of human APP and, in the C-terminal direction, up to 30 of amino acid residues 327 to 328 of human APP, the whole forming a reverse-order sequence relative to human APP.

In formula (VII), X₁ is preferably from zero to 20 and/or X₂ is from zero to 20. More preferably, X₁ is from zero to 10 and/or X₂ is from zero to 10. Still more preferably, X₁ and/or X₂ is zero.

In other preferred compounds of formula (VII), X₁ is 2 or less and X₂ is 8 or less.

### BRIEF DESCRIPTION OF THE DRAWINGS

The drawings of this specification consist of the following:
Figure 1 which shows the amino acid sequence of human APP and chick APP, as referred to hereinbefore;
Figure 2 shows the effect of β-amyloid 12-28 polypeptide on memory formation;
Figure 3 shows the effect of RERMS on β-amyloid 12-28 induced amnesia;
Figure 4 shows the effect of RERMS on anti-APP induced amnesia;
Figure 5 shows the effect of RERMS, SMRER and RSAER on APP-antisense induced amnesia;
Figure 6 shows the effect of APP 319-335 on APP-antisense induced amnesia;
Figure 7 shows the effect of RERMS on weak training;
Figure 8 shows the effect of APP 319-335 on weak training;
Figure 9 shows the effect of RER on weak training;
Figure 10 shows the effects of RER and Ac-RER on β-amyloid 12-28 induced amnesia;
Figure 11 shows the effect of Ac-RER on weak training;
Figure 12 shows the effects of differing doses of RER and Ac-RER; and
Figure 13 shows the effect of Ac-RER on β-amyloid 12-28 induced amnesia.

### DETAILED DESCRIPTION OF THE INVENTION

The invention will now be described further by way of example with reference to the following experimental procedures and results.

### Materials and methods

### Animals and training

Commercially obtained Ross Chunky eggs were incubated and hatched in brooders and held until 16 ±6 hours old. Chicks were placed in pairs in small aluminium pens. Following an equilibration period of an hour, the chicks were pretrained and trained essentially as described by Lossner and Rose (J. Neurochem. 41, 1357-1363 (1983), the contents of which are incorporated herein by reference). Pretraining involved three 10 s presentations of a small (2 mm diameter) white bead, at approximately 5 minute intervals. Chicks, which failed to peck the bead at least twice in three presentations (less than 5%), were not used subsequently, but remained in their pens for the duration of the experiment. Two training techniques were used: "strong" and "weak" training. In both, 5 to 10 minutes after the last pre-training trial, chicks were trained by a 10 s presentation of a 4 mm diameter chrome bead, which had been dipped in the bitter-tasting methylanthranilate. Control chicks pecked at a water-coated or dry bead. In the "strong" version of the task, 100% methylanthranilate was used. In the "weak" version, 10% methylanthranilate was used. Chicks spontaneously pecked at the training or control beads within 20 s. Chicks that peck at the bitter bead evinced a disgust reaction and would not normally peck at a similar, but dry bead for some hours subsequently. At various times following training chicks were tested, by offering them a dry 4 mm diameter chrome bead, followed 10 minutes later by a small (2 mm diameter) white bead, each for 20 to 30 s. Animals were tested by an experimenter blind as to which treatment each chick had received. Chicks are considered to remember the task if they avoid the chrome bead at test but peck at the white bead (discriminate), and to have forgotten it if they peck at both beads. Recall is calculated as a percent avoidance score(percentage of chicks which avoid the chrome bead) and as a discrimination score (percentage of chicks which avoid the chrome but peck at the white bead). The use of the discrimination score ensures that chicks can indeed see and peck accurately at the bead; and hence that the avoidance of the chrome bead is not due to non-specific factors such as lack of visuo-motor coordination, motivation, attention, arousal, etc. but is a positive act, demonstrating memory for the distasteful stimulus. Each chick was trained and tested only once and differences between groups tested for statistical significance by g-test described by Sokal and Rohlf (Biometry: the Principles and Practice of Statistics in Biological Research (2nd edition), W H Freeman, New York (1981)), the contents of which are incorporated herein by reference. The validity of this particular training task used to assess memory formation is extensively discussed by Andrew (Neural and Behavioural Plasticity: the Use of the Domestic Chick as a Model, Oxford University Press, Oxford, UK (1991), the contents of which are incorporated herein by reference.

Chicks trained on the strong version of the task were found to recall the avoidance for at least 48 hours, and more than 80% were found normally to avoid and discriminate on test at 24 hours. Therefore if agents that are amnesic - that is, cause the chick not to remember - are administered, chicks will demonstrate forgetting by pecking rather than avoiding the chrome bead on test. By contrast, chicks were found normally to remember the "weak" version of the task for only a few hours - some 6 to 8 hours in all; retention at 24 hours was normally reduced to some 20 to 30%. Thus the learning experience is not committed to long-term memory. Agents that are memory enhancers can thus be tested. A memory enhancing agent, administered to a chick trained on the weak learning task, produces an increase in retention - increased avoidance of the chrome bead - at 24 hours. That is, such memory enhancers help convert weak to strong learning by enabling the transition from shorter to longer-term memory.

### Peptide injections

Bilateral intracranial injections (2 µg/hemisphere) of either saline, or solutions in saline of different peptides (0.5 to 5 µg/hemisphere) homologous to different regions of the external domain of human APP were injected intracerebrally into a specific brain region, known to be required for memory formation (the intermediate hyperstriatum ventrale) at different time-points pre- or post-training using a 5µg Hamilton syringe fitted with a plastic sleeve to allow a penetration of 3 mm. After completion of the injection, the needle was kept in place for 5 s. Correct placement was ensured by using a specially designed headholder described by Davis et al (Physiol. Behav. 22, 177-184 (1979), the contents of which are incorporated herein by reference) and was routinely visually monitored postmortem. Peptides or other substances were administered at various times either before or after the training protocol. Chicks were tested at different time points post-training as described above.
The general behaviour of the chicks following injections was observed to detect any potential non-specific or adverse reactions to the injections.

### Peptide Materials

The polypeptides administered were synthesised using a conventional peptide synthesiser in a manner which is well-known to those skilled in the art. The synthesised polypeptides were purified by use of RP-HPLC and purity further checked by mass spectrometry (MALDI-TOF), both techniques being well known to those skilled in the art. The polypeptides after synthesis were kept under argon in a lyophilised state, the argon preventing oxidation of cysteine, methionine and tryptophan in particular.

Polypeptide synthesis as just mentioned is carried out by MWG-Biotech UK Limited of Milton Keynes, UK.

RERMS is also available from Bachem Limited of St. Helens, Merseyside, UK.

Ac-RER can be synthesised by techniques well-known to those skilled in the art. It was obtained from Cambridge Research Biochemicals Limited of Billingham, Cleveland, UK.

For further details regarding synthetic methods for the preparation of peptides and peptide derivatives, reference is made to "Principles of Peptide Synthesis" by M. Bodanszky, 2nd Edition (Springer Laboratory, 1993), the entire contents of which are incorporated herein by reference.

### Experimental results

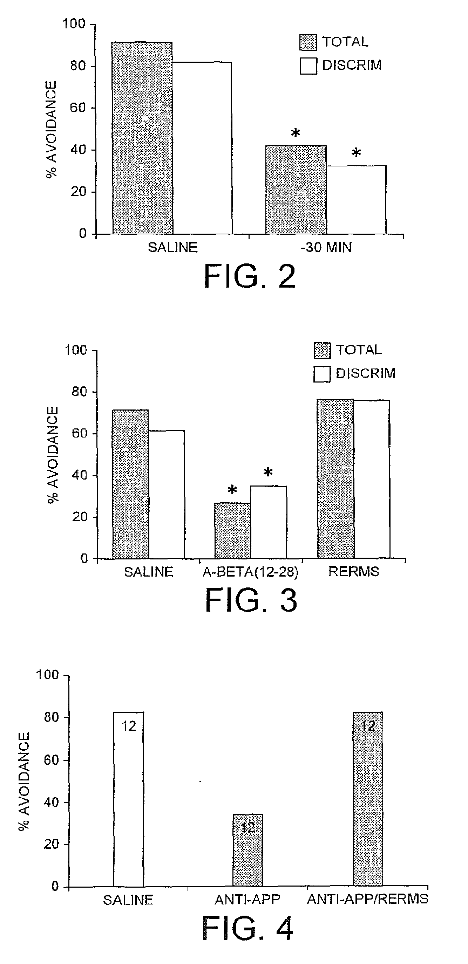

It is well known in many animal model systems for the study of memory that injection of β-amyloid and β-amyloid peptides, such as β-amyloid 12-28, results in a failure of animals to retain recently acquired memories. Figure 2 shows this result for a chick; injection of β-amyloid 12-28 into the brain 30 minutes prior to training chicks on the passive avoidance task results in amnesia in animals tested 30 minutes subsequently.

Figure 2 shows in the left-hand half the percent avoidance measured in terms of total avoidance and discrimination for a saline control and in the right-hand half the percent avoidance measured when β-amyloid 12-28 is injected as described above 30 minutes pretraining and memory is tested 30 minutes posttraining.

However, if amnesia is induced by injection of β-amyloid 12-28 30 minutes pretraining, and RERMS is injected 20 minutes pretraining, memory retention is restored. Figure 3 shows that in this case memory is normal at 24 hours post-training.

Figure 3 shows on the left the percent avoidance measured in terms of total avoidance and discrimination for a saline control, in the centre the corresponding results when β-amyloid 12-28 is injected 30 minutes pretraining and memory tested 24 hours posttraining, and on the right the results when the pretraining injection of β-amyloid 12-28 is followed 10 minutes later by RERMS and memory is again tested 24 hours posttraining.

It is thus shown that RERMS can prevent the memory loss produced by β-amyloid 12-28, a component of the amyloid plaques characteristic of Alzheimer's disease.

It is known that disrupting the normal function of APP by blocking its external domain with a specific monoclonal antibody (mb22C11) around the time of training, whilst without effect on the ability of chicks to learn the passive avoidance response, prevents the transition to long term memory. The monoclonal antibody mb22C11, available from Boehringer-Mannheim, specifically recognises an epitope consisting of part of the external domain of APP.

Figure 4 shows on the left the percent avoidance measured for chicks injected with a saline control, in the centre the percent avoidance measured when mb22C11 is injected (1-5 µg in 2 µl) intracerebrally as described hereinbefore for peptide injections 30 minutes pretraining and, on the right, the percent avoidance measured when RERMS is also injected 25 minutes after mb22C11 (5 minutes pretraining). In all cases, memory was tested 24 hours posttraining.

The results shown in figure 4 demonstrate that RERMS injected 5 minutes before training will prevent antibody induced memory loss and that the peptide RERMS can prevent anti-APP induced memory loss. Thus, RERMS can prevent the memory loss resulting from disrupting the normal function of APP.

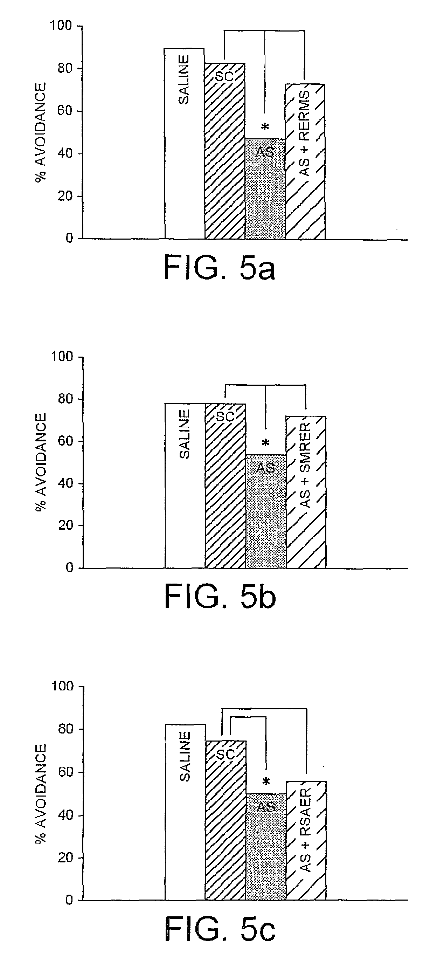

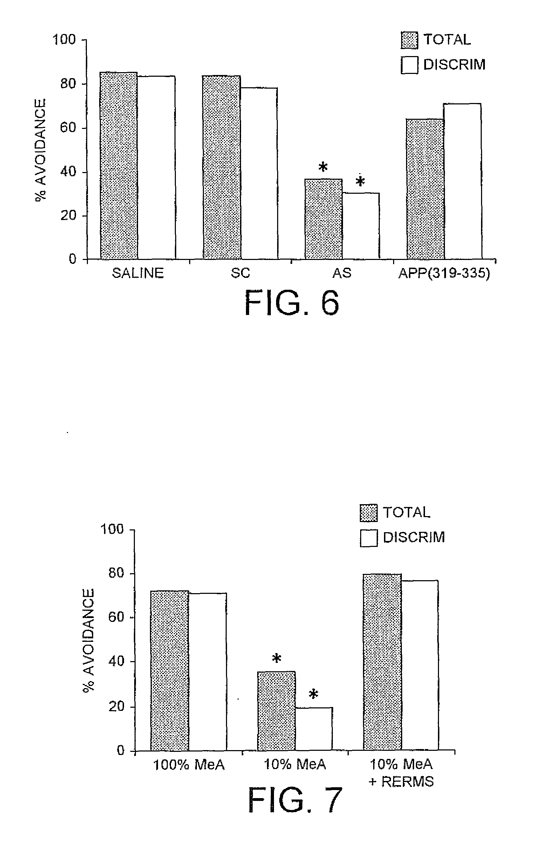

Figures 5 and 6 show the effect of inducing memory loss by injection of a 16-mer end-protected phosphodiester oligodeoxynucleotide designed to correspond to the transcription start sites 146 and AUG₁₇₈₆ of the APP mRNA, immediately upstream of a ribozyme binding site. The oligodeoxynucleotide, 5'-CCC GAG GAC TGA GCC A-3' (SEQ ID No: 21) was further modified on the 2nd and 13th nucleotides to prevent internal looping and is available from King's College Molecular Medicine Unit, London, UK. The oligodeoxynucleotide was used in scrambled (SC) and antisense (AS) forms and administered as described hereinbefore for peptide administration in an amount of 0.6 to 1.0 µg in 2 µl.

In a first experiment, chicks were injected with saline, RERMS, SMRER and RSAER in various combinations in the amounts stated hereinbefore.

The results are shown in figure 5 which shows the percent avoidances measured on the "strong" learning task described hereinbefore. Figure 5a shows the effect compared with a saline control of administration separately of SC oligodeoxynucleotide 12 hours pretraining and AS oligodeoxynucleotide 12 hours pretraining, together with the effect of administration of RERMS following the AS oligodeoxynucleotide 30 minutes pretraining.

Figure 5a shows that the SC oligodeoxynucleotide had no effect on memory but the AS compound had a significant effect of memory loss which was avoided to a substantial extent when RERMS was administered.

Figure 5b shows that similar results were obtained with the reverse-order pentapeptide SMRER.

Figure 5c shows that the effect obtained with RERMS and SMRER is absent with the pentapeptide RSAER.

In a second experiment, SC and AS oligodeoxynucleotides were administered 12 hours pretraining. A polypeptide (APP 319-335) corresponding to amino acid residues 319 to 335 of human APP was injected 30 minutes pretraining. Chicks were tested for memory according to the "strong" version of the test described hereinbefore 30 minutes posttraining.

Figure 6 shows successively from the left: the percent avoidance measured for a saline control; the percent avoidance measured when SC oligodeoxynucleotide was administered; the percent avoidance measured when AS oligodeoxynucleotide was administered; and the percent avoidance measured when APP 319-335 was administered 30 minutes pretraining following administration of AS oligodeoxynucleotide 12 hours pretraining. Each result is shown both in terms of total avoidance (left-hand column) and discrimination (right-hand column).

The results shown in figure 6 demonstrate that APP319-335 can prevent antisense induced memory loss.

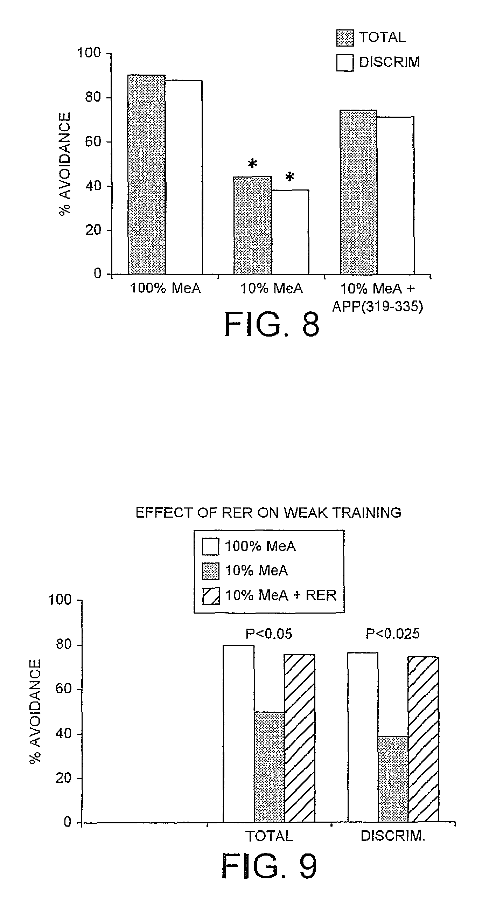

Figures 7 and 8 show the effects of RERMS and APP 319-335 on memory in chicks trained on the "weak" memory test described hereinbefore.

As stated hereinbefore, weakly trained chicks (trained on 10% methylanthranilate) retain memory for the avoidance for only some 6 hours, and thereafter forget. Figure 7 shows, on the left, the percent avoidance results (in terms of total avoidance and discrimination) for chicks trained on the "strong" version of the training, in the centre the corresponding results for "weak" training and, on the right, the effect of administration of RERMS following "weak" training. The chicks were tested for memory 24 hours posttraining; RERMS was administered in accordance with the procedure described hereinbefore 30 minutes pretraining.

Figure 8 shows the corresponding results obtained when the APP 319-335 polypeptide was used instead of RERMS.

Figures 7 and 8 show that RERMS and APP 319-335 if injected prior to training chicks on the weak task, enhance memory at 24 hours. They thus function as cognitive enhancers (nootropic agents). Thus, RERMS and APP 319-335 both enhance normal memory in weakly trained animals.

Figure 9 shows the effect of RER on memory in chicks trained on the "weak" memory test described hereinbefore.

As stated hereinbefore, weakly trained chicks (trained on 10% methylanthranilate) retain memory for the avoidance for only some 6 hours, and thereafter forget.

Figure 9 shows, in the three columns on the left, on the left the percent avoidance results (in terms of total avoidance) for chicks trained on the "strong" version of the training, in the centre the corresponding results for "weak" training and, on the right, the effect of administration of RER following "weak" training. The chicks were tested for memory 24 hours posttraining; RER was administered in accordance with the procedure described hereinbefore 30 minutes pretraining.

Figure 9 shows, on the right, the corresponding data in terms of discrimination.

Figure 9 shows that RER, if injected prior to training chicks on the weak task, enhances memory at 24 hours. It thus functions as a cognitive enhancer (nootropic agent). Thus, RER enhances normal memory in weakly trained animals.

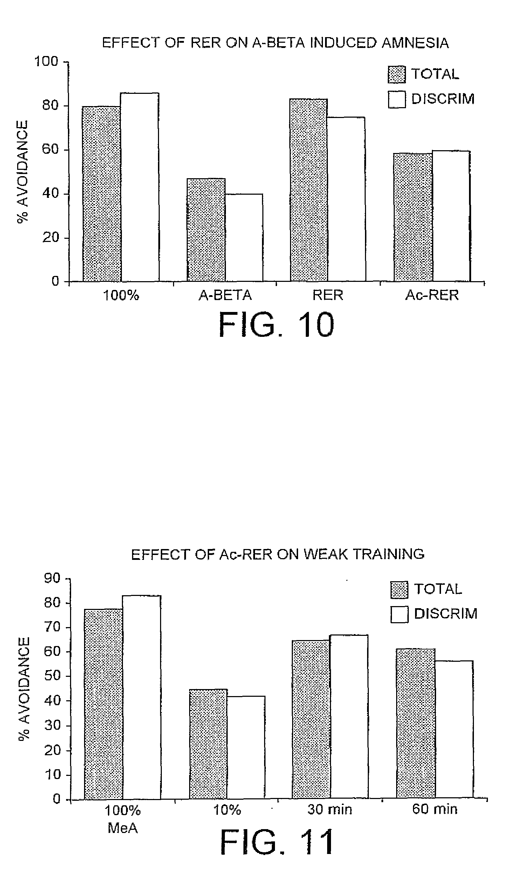

Figure 10 shows the effect of RER and Ac-RER on β-amyloid 12-28 induced amnesia in chicks trained on the "strong" memory test described hereinbefore.

Figure 10 shows from left to right the percent avoidance measured in terms of total avoidance and discrimination for
- chicks trained on the "strong" version of the training;
- the amnesic effect of β-amyloid 12-28 administered at 2 µg/hemisphere 30 mins prior to training;
- the effect of RER administered at 2 µg/hemisphere 20 mins prior to training after administration of β-amyloid 12-28 30 mins prior to training; and
- the effect of Ac-RER administered at 2 µg/hemisphere 20 mins prior to training after administration of β-amyloid 12-28 30 mins prior to training.

The results in figure 10 show that both RER and Ac-RER have the effect of restoring memory measured 20 mins after administration of RER or Ac-RER, following amnesia induced by administration of β-amyloid 12-28.

The results also show that the memory-restorative effect of Ac-RER is less than that of RER but indicate that Ac-RER is more stable after administration and therefore more suitable for peripheral administration.

Figure 11 shows the effect of Ac-RER on memory in chicks trained on the "weak" memory test described hereinbefore.

Figure 11 shows from left to right the percent avoidance measured in terms of total avoidance and discrimination for
- chicks trained on the "strong" version of the training:
- chicks trained on the "weak" version of the training;
- chicks trained on the "weak" version of the training after having had Ac-RER administered at 2 µg/hemisphere 30 mins beforehand; and
- chicks trained on the "weak" version of the training after having had Ac-RER administered at 2 µg/hemisphere 60 mins beforehand.

The results in figure 11 show that Ac-RER has the effect of improving the memory of weakly trained chicks when administered 30 or 60 minutes beforehand. The results also show that the effect 60 minutes after administration is still significant, although less than 30 minutes after administration.

The results therefore show that Ac-RER has the effect of a cognitive enhancer and that the effect is apparent over a significant time period.

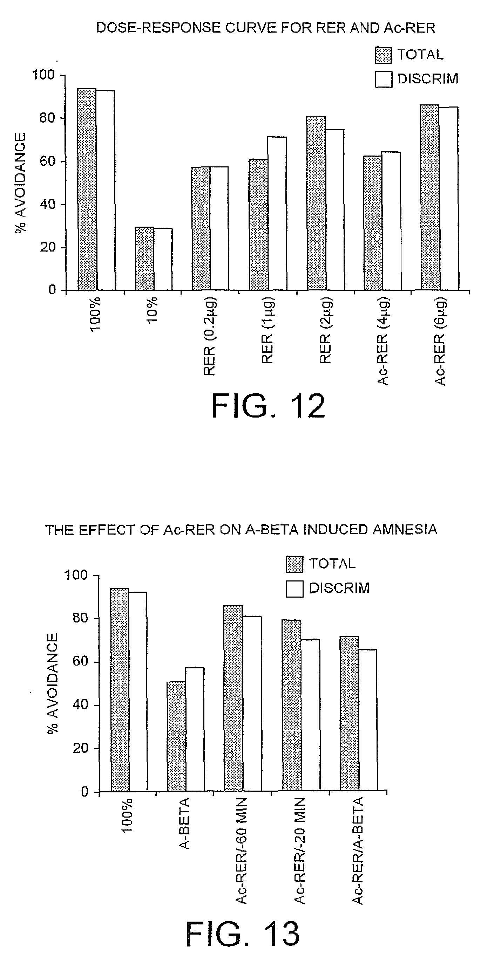

Figure 12 shows the effect of different doses of RER and Ac-RER on memory in chicks trained on the "weak" memory test described hereinbefore.

Figure 12 shows from left to right the percent avoidance measured in terms of total avoidance and discrimination for
- chicks trained on the "strong" version of the training;
- chicks trained on the "weak" version of the training;
- chicks trained on the "weak" version of the training after having had RER administered 30 mins beforehand at 0.2 µg/hemisphere;
- chicks trained on the "weak" version of the training after having had RER administered 30 mins beforehand at 1.0 µg/hemisphere;
- chicks trained on the "weak" version of the training after having had RER administered 30 mins beforehand at 2.0 µg/hemisphere;
- chicks trained on the "weak" version of the training after having had Ac-RER administered 30 mins beforehand at 4.0 µg/hemisphere; and
- chicks trained on the "weak" version of the training after having had Ac-RER administered 30 mins beforehand at 6.0 µg/hemisphere.

The results in figure 12 show that both RER and Ac-RER have the effect of improving the memory of weakly-trained chicks when administered 30 minutes beforehand and that higher doses of Ac-RER than of RER are required to obtain a comparable effect.

The results therefore show that increased amounts of Ac-RER, in comparison to RER, are required to be used as a cognitive enhancer. This again indicates that Ac-RER is more stable after administration than RER and therefore more suitable for peripheral administration.

Figure 13 shows the effect of Ac-RER administered at different times on β-amyloid 12-28 induced amnesia in chicks trained on the "strong" memory test described hereinbefore.

Figure 13 shows from left to right the percent avoidance measured in terms of total avoidance and discrimination for
- chicks trained on the "strong" version of the training;
- the amnesic effect of β-amyloid 12-28 administered at 2 µg/hemisphere 30 mins prior to training;
- the memory-restorative effect of Ac-RER administered at 4 µg/hemisphere 60 mins prior to training and 30 mins prior to administration of β-amyloid 12-28 at 2 µg/hemisphere;
- the memory-restorative effect of Ac-RER administered at 4 µg/hemisphere 20 mins prior to training and 10 mins after administration of β-amyloid 12-28 at 2 µg/hemisphere; and
- the memory-restorative effect of Ac-RER administered at 4 µg/hemisphere together with β-amyloid 12-28 at at 2 µg/hemisphere 30 mins prior to training.

The results of figure 13 show that Ac-RER has the effect of restoring memory when amnesia has been induced by β-amyloid 12-28. The effect is manifested regardless of whether Ac-RER is administered before, after or simultaneously with the amnesia-inducing β-amyloid 12-28.

The role of APP in memory formation has been attributed to its involvement in cell-to-substrate adhesion processes. The data reported suggests that the APP involvement in memory formation most probably involves change in signal transduction events. The post-training time within which the antibody and antisense-induced amnesia, and within which RER, Ac-RER, RERMS and SMRER prevents amnesia, corresponds to that during which memory formation is vulnerable to disruption of the putative signal-transduction functions of APP.

The chick system is a good one for exploring these issues, because the learning task is precise and sharply timed, and permits one also to be sure that any observed effect of an injected substance is specific to retention and not either to acquisition or to concomitant processes such as visual acuity, arousal or motor activity. Further, the role of other cell adhesion molecules in the cascade leading to synaptic modulation has been well mapped, so that the effects of either blocking or attempting to rescue functional APP activity can be set into an established context : see Rose, Learn. Memory 7, 1-17 (2000) the contents of which are fully incorporated herein by reference.

It is therefore indicated by the experimental results reported above that compounds of the present invention are effective for the treatment and/or prevention of neurological diseases and disorders and as cognitive enhancers (nootropic agents) in other animals, including human and non-human mammals. The compounds are therefore effective in the treatment and/or prevention of Alzheimer's disease in humans and other neurodegenerative diseases and disorders in animals generally, including humans. Such animals include transgenic and other animal models for Alzheimer's disease.

As used herein, except where the context indicates otherwise, the terms "treatment", "treat" and analogous expressions used in relation to neurodegenerative diseases include within their scope not only treatment when symptoms are apparent but also the partial or total prevention of such diseases and delay in their onset in patients or animals who are subjected to treatment before onset of the disease or its symptoms become apparent.

The compounds of the present invention may be administered intracerebally as described above, or may be administered peripherally, for example intramuscularly, intravenously, transdermally or orally, preferably after complexation as described above. Instead or in addition, the compounds may be protected against alteration between administration and effectiveness, for example by addition of protective groups.

The experimental results of figures 10 to 13 show that the polypeptide derivatives of the present invention, particularly Ac-RER, are effective for longer time periods after administration than their analogues lacking protective groups and are therefore particularly suitable for peripheral administration.

The compounds of the present invention may also be formulated as pharmaceutical compositions as referred to hereinbefore, particularly such compositions as are capable of crossing the blood-brain barrier and thereby be suitable for peripheral administration.

In all events a suitable dose of peptide compounds according to the invention is from 10 to 100 µg/kg body weight of the animal being treated.

As used herein, the term "effective to treat" in the context of a neurodegenerative disease means that amount of the compound(s) used in the treatment which causes a reduction or stabilisation or, as the case may be, prevents or delays the appearance of such symptoms as measured by standard medical or psychological criteria, for example as disclosed in Handbook of Memory Disorders (eds: A D Baddeley, B A Wilson and F N Watts), Wiley (1995), the disclosure of which is herein incorporated by reference.

As used herein, the term "effective to treat" in relation to a cognitive enhancement means an amount of the compound(s) used in the treatment which causes an improvement in cognitive power as measured by psychological criteria, for example as disclosed in Handbook of Memory Disorders (eds: A D Baddeley, B A Wilson and F N Watts), Wiley (1995), the disclosure of which is herein incorporated by reference.

### SEQUENCE LISTING FREE TEXT

| SEQ ID NO. | Free Text <223> |
|---|---|
| 3 | Description of Artificial Sequence: 5-mer polypeptide |
| 4 | Description of Artificial Sequence: 5-mer polypeptide |
| 5 | Description of Artificial Sequence: 5-mer polypeptide |
| 6 | Description of Artificial Sequence: 17-mer polypeptide |
| 7 | Description of Artificial Sequence: 17-mer polypeptide |
| 8 | Description of Artificial Sequence: 17-mer polypeptide |
| 9 | Description of Artificial Sequence: 3-mer polypeptide |
| 10 | Description of Artificial Sequence: 4-mer polypeptide |
| 11 | Description of Artificial Sequence: 4-mer polypeptide |
| 12 | Description of Artificial Sequence: 16-mer polypeptide |
| 13 | Description of Artificial Sequence: 15-mer polypeptide |
| 14 | Description of Artificial Sequence: 14-mer polypeptide |
| 15 | Description of Artificial Sequence: 12-mer polypeptide |
| 16 | Description of Artificial Sequence: 11-mer polypeptide |
| 17 | Description of Artificial Sequence: 11-mer polypeptide |
| 18 | Description of Artificial Sequence: 10-mer polypeptide |
| 19 | Description of Artificial Sequence: 6-mer polypeptide |
| 20 | Description of Artificial Sequence: 22-mer polypeptide |
| 21 | Description of Artificial Sequence: end-protected 16-mer oligonucleotide modified at positions 2 & 13 to prevent internal looping |
| 22 | Description of Artificial Sequence: 40-mer polypeptide |

Aspects of the disclosure of the present application are defined in the following clauses:
1. A derivative of a polypeptide having the formula:

X₁-Arg-Xaa-Arg-X₂ (I)

wherein X₁ and X₂, which may be the same or different, each represents from zero to 30 natural or synthetic amino acid residues or derivatives thereof and Xaa represents a natural or synthetic amino acid residue or a derivative thereof, at least one functional group of at least one said amino acid residue or derivative thereof being protected by a protective group.
2. A polypeptide derivative according to clause 1, in which the protected functional group(s) comprise one or more amino groups.
3. A polypeptide derivative according to clause 2, in which the amino group(s) comprise an N-terminal amino group.
4. A polypeptide derivative according to clause 2 or 3, in which at least one protected amino group is protected by replacement of only one of its hydrogen atoms by a protective group.
5. A polypeptide derivative according to clause 1, in which the protected functional group(s) comprise one or more carboxyl groups.
6. A polypeptide derivative according to clause 4, in which the carboxyl group(s) comprise a C-terminal carboxyl group.
7. A polypeptide derivative according to any preceding clause, in which at least one protective group is an acyl group represented by the formula 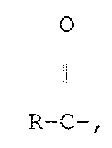 wherein R represents a substituted or unsubstituted straight- or branched-chain alkyl group, a substituted or unsubstituted cycloalkyl group, a substituted or unsubstituted straight or branched-chain aralkyl group or a substituted or unsubstituted aryl group, the groups R being the same or different when both said hydrogen are replaced by acyl groups.
8. A polypeptide derivative according to clause 7, in which R represents a C₁ to C₆ straight- or branched-chain alkyl group.
9. A polypeptide derivative according to clause 8, in which R represents a methyl group.
10. A polypeptide derivative according to any preceding clause, in which X₁ is from zero to 20 and/or X₂ is from zero to 20.
11. A polypeptide derivative according to clause 7, in which X₁ is from zero to 10 and/or X₂ is from zero to 10.
12. A polypeptide derivative according to clause 8, in which X₁ and/or X₂ is zero.
13. A polypeptide derivative according to any of clauses 1 to 9, in which X₁ and X₂ are such that formula (I) represents an amino acid sequence which is identical or closely homologous to amino acid residues 328 to 330 of human APP and up to 30 successive amino acid residues of human APP extending in each direction therefrom, or to a sequence which is closely homologous thereto or in which the amino acids thereof are replaced by nonstandard amino acids.
14. A polypeptide derivative according to any of clauses 1 to 9, in which X₁ and X₂ are such that formula (I) represents a reverse-order amino acid sequence which is identical or closely homologous to amino acid residues 330 to 328 of human APP and up to 30 successive amino acid residues of human APP extending in each direction therefrom, or to a sequence which is closely homologous thereto or in which the amino acids thereof are replaced by nonstandard amino acids.
15. A polypeptide derivative according to any of clauses 1 to 9, in which X₁ represents:

X₃-Ala-Lys-Glu-Arg-Leu-Glu-Ala-Lys-His

wherein X₃ represents from zero to 21 natural or synthetic amino acid residues or derivatives thereof, and/or X₂ represents:

Met-Ser-Gln-Val-Met-X₄

wherein X₄ represents from zero to 25 natural or synthetic amino acid residues or derivatives thereof.
16. A polypeptide derivative according to clause 15, in which X₃ is from zero to 20 and/or X₄ is from zero to 20.
17. A polypeptide derivative according to clause 16, in which X₃ is from zero to 10 and/or X₄ is from zero to 10.
18. A polypeptide derivative according to clause 17, in which X₃ and/or X₄ is zero.
19. A polypeptide derivative according to any of clauses 1 to 9, in which X₁ represents:

X₃-Met-Val-Gln-Ser-Met

wherein X₃ represents from zero to 25 natural or synthetic amino acid residues or derivatives thereof, and/or X₂ represents:

His-Lys-Ala-Glu-Leu-Arg-Glu-Lys-Ala-X₄

wherein X₄ represents from zero to 21 natural or synthetic amino acid residues or derivatives thereof.
20. A polypeptide derivative according to clause 19, in which X₃ is from zero to 20 and/or X₄ is from zero to 20.
21. A polypeptide derivative according to clause 20, in which X₃ is from zero to 10 and/or X₄ is from zero to 10.
22. A polypeptide derivative according to clause 21, in which X₃ and/or X₄ is zero.
23. A polypeptide derivative according to any of clauses 1 to 9, in which X₂ represents:

Met - X₄

wherein X₄ represents from zero to 29 natural or synthetic amino acid residues or derivatives thereof.
24. A polypeptide derivative according to clause 23, in which X₂ represents:

Met-Ser-X₄

wherein X₄ represents from zero to 28 natural or synthetic amino acid residues or derivatives thereof.
25. A polypeptide derivative according to any of clauses 1 to 9, in which X₁ represents:

X₃-Met

wherein X₃ represents from zero to 29 natural or synthetic amino acid residues or derivatives thereof.
26. A polypeptide derivative according to clause 25, in which X₁ represents:

X₃-Ser-Met

wherein X₃ represents from zero to 28 natural or synthetic amino acid residues or derivatives thereof.
27. A polypeptide derivative according to any of clauses 1 to 9, in which Xaa is glutamic acid, X₁ is methionine and X₂ is zero.
28. A polypeptide derivative according to any of clauses 1 to 9, in which Xaa is glutamic acid, X₁ is zero and X₂ is methionine.
29. A polypeptide derivative according to any of clauses 1 to 9, in which Xaa is glutamic acid and X₁ and X₂ are both zero.
30. A polypeptide derivative according to any of clauses 1 to 9, in which Xaa is glutamic acid, X₁ is zero and X₂ is Met-Ser.
31. A polypeptide derivative according to any of clauses 1 to 9, in which Xaa is glutamic acid, X₁ is Ser-Met and X₂ is zero.
32. A compound having the formula 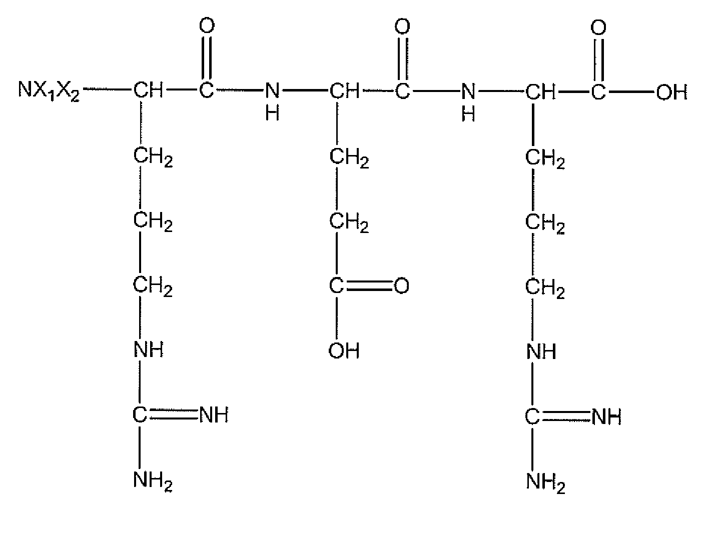 in which X₁ is a protective group and X₂ is H or a protective group.
33. A compound having the formula 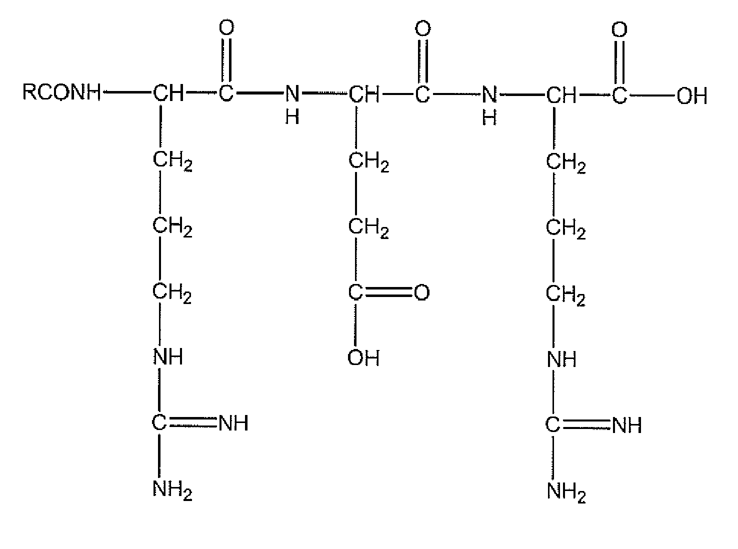 in which R represents a substituted or unsubstituted straight- or branched-chain alkyl group, a substituted or unsubstituted cycloalkyl group, a substituted or unsubstituted straight or branched-chain aralkyl group or a substituted or unsubstituted aryl group.
34. A compound according to clause 33, in which R represents a C₁ to C₆ straight- or branched-chain alkyl group.
35. A compound having the formula 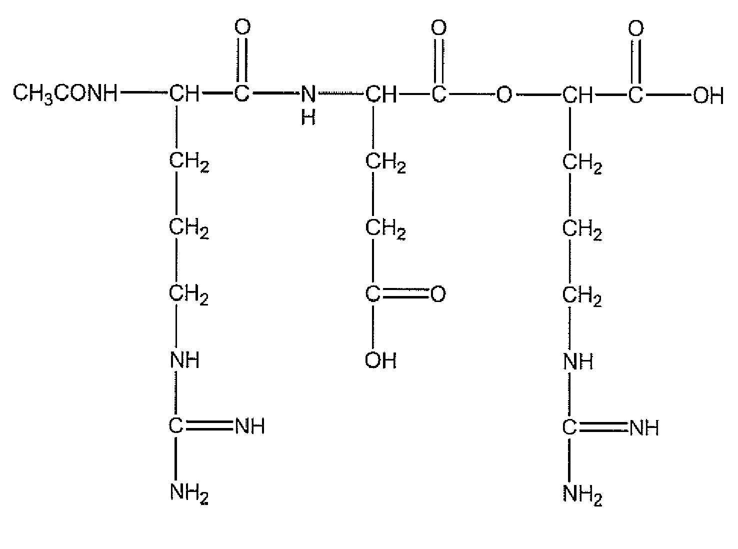 36. A polypeptide derivative, according to any of clauses 1 to 31 or a compound according to any of clauses 32 to 35 for use in medicine.
37. Use of a polypeptide derivative according to any of clauses 1 to 31 or a compound according to any of clauses 32 to 35 in the preparation of a medicament for use in the treatment of a neurodegenerative disease or as a cognitive enhancer.
38. Use according to clause 37, in which the neurodegenerative disease is Alzheimer's disease.
39. A pharmaceutical composition comprising a polypeptide derivative according to any of clauses 1 to 31 or a compound according to any of clauses 32 to 35 and a pharmaceutically acceptable carrier.
40. A pharmaceutical composition in which a polypeptide derivative according to any of clauses 1 to 31 or compound according to any of clauses 32 to 35 is chemically or physically linked to a further molecule or vehicle to form a complex for pharmaceutical delivery of the said compound.
41. A method of treatment of a neurodegenerative disease in an animal, comprising:
identifying an animal suffering or potentially suffering from a neurodegenerative disease; and
administering to the animal an amount of a polypeptide derivative according to any of clauses 1 to 31, of a compound according to any of clauses 32 to 35, or of a composition according to clause 39 or 40 effective treat the neurodegenerative disease.
42. A method according to clause 41, in which the neurodegenerative disease is Alzheimer's disease.
43. A method of producing a cognitive enhancement effect in an animal, comprising:
providing an animal in which the effect is to be produced; and
administering to the animal an amount a polypeptide derivative according to any of clauses 1 to 31, of a compound according to any of clauses 32 to 35, or of a composition according to clause 39 or 40 effective to produce the cognitive enhancement.
44. A compound having a formula comprising:

X₁-Arg-Glu-Arg-X₂ (II)

wherein X₁ and X₂, which may be the same or different, each represents from zero to 32 natural or synthetic amino acid residues or derivatives thereof, and Xaa represents a natural or synthetic amino acid residue or a derivative thereof, provided that the compound is none of the following (as hereinbefore defined): APP 296-335, APP 319-335, APP 317-332, APP 321-335, APP 319-332, APP 321-332, APP 321-331, APP 325-335, APP 321-330, APP 327-332, APP 328-332, APP 328-331, APP 328-330, APP 335-319 and APP 316-337.
45. A compound according to clause 44, in which X₁ is from zero to 22 and/or X₂ is from zero to 22.
46. A compound according to clause 45, in which X₁ is from zero to 12 and/or X₂ is from zero to 12.
47. A compound according to clause 46, in which either X₁ or X₂ is zero.
48. A compound according to clause 44, in which X₁ and X₂ are such that formula (II) represents an amino acid sequence which is identical or closely homologous to amino acid residues 328 to 330 of human APP and up to 32 successive amino acid residues of human APP extending in each direction therefrom, the formula also comprising sequences closely homologous to said sequence and sequences in which said amino acids thereof are replaced by nonstandard amino acids and/or by derivatives of acid amino acids.
49. A compound according to clause 44, in which X₁ and X₂ are such that formula (II) represents a reverse-order amino acid sequence which is identical or closely homologous to amino acid residues 330 to 328 of human APP and up to 32 successive amino acid residues of human APP extending in each direction therefrom, the formula also comprising sequences closely homologous to said reverse-order sequence and sequences in which said amino acids thereof are replaced by nonstandard amino acids and/or by derivatives of acid amino acids.
50. A compound having a formula comprising:

X₁-Arg-Xaa-Arg-X₂ (III)

wherein X₁ and X₂, which may be the same or different, each represents from zero to 32 natural or synthetic amino acid residues or derivatives thereof and Xaa represents a natural or synthetic amino acid residue or a derivative thereof, for use in medicine.
51. A compound according to clause 50, in which X₁ is from zero to 22 and/or X₂ is from zero to 22.
52. A compound according to clause 51, in which X₁ is from zero to 12 and/or X₂ is from zero to 12.
53. A compound according to clause 52, in which X₁ and/or X₂ is zero.
54. A compound according to clause 50, in which X₁ and X₂ are such that formula (III) represents an amino acid sequence which is identical or closely homologous to amino acid residues 328 to 330 of human APP and up to 32 successive amino acid residues of human APP extending in each direction therefrom, the formula also comprising sequences closely homologous to said sequence and sequences in which said amino acids thereof are replaced by nonstandard amino acids and/or by derivatives of acid amino acids.
55. A compound according to clause 50, in which X₁ and X₂ are such that formula (III) represents a reverse-order amino acid sequence which is identical or closely homologous to amino acid residues 330 to 328 of human APP and up to 30 successive amino acid residues of human APP extending in each direction therefrom, the formula also comprising sequences closely homologous to said reverse-order sequence and sequences in which said amino acids thereof are replaced by nonstandard amino acids and/or by derivatives of acid amino acids.
56. A compound according to clause 50, in which X₁ represents:

X₃-Ala-Lys-Glu-Arg-Leu-Glu-Ala-Lys-His

wherein X₃ represents from zero to 23 natural or synthetic amino acid residues or derivatives thereof, and/or X₂ represents:

Met-Ser-Gln-Val-Met-X₄

wherein X₄ represents from zero to 27 natural or synthetic amino acid residues or derivatives thereof.
57. A compound according to clause 56, in which X₃ is from zero to 20 and/or X₄ is from zero to 20.
58. A compound according to clause 57, in which X₃ is from zero to 10 and/or X₄ is from zero to 10.
59. A compound according to clause 58, in which X₃ and/or X₄ is zero.
60. A compound according to clause 50, in which X₁ represents:

X₃-Met-Val-Gln-Ser-Met

wherein X₃ represents from zero to 27 natural or synthetic amino acid residues or derivatives thereof, and/or X₂ represents:

His-Lys-Ala-Glu-Leu-Arg-Glu-Lys-Ala-X₄

wherein X₄ represents from zero to 23 natural or synthetic amino acid residues or derivatives thereof.
61. A compound according to clause 60, in which X₃ is from zero to 20 and/or X₄ is from zero to 20.
62. A compound according to clause 61, in which X₃ is from zero to 10 and/or X₄ is from zero to 10.
63. A compound according to clause 62, in which X₃ and/or X₄ is zero.
64. A compound according to any of clauses 1 to 26 and 44 to 63, in which Xaa is glutamic acid.
65. A compound according to clause 50, in which Xaa is glutamic acid, X₁ is methionine and X₂ is zero.
66. A compound according to clause 50, in which Xaa is glutamic acid, X₁ is zero and X₂ is methionine.
67. A compound according to clause 50, in which Xaa is glutamic acid, and X₁ and X₂ are both zero.
68. Use of a compound having a formula comprising:

X₁-Arg-Xaa-Arg-X₂ (III)

wherein X₁ and X₂, which may be the same or different, each represents from zero to 32 natural or synthetic amino acid residues or derivatives thereof and Xaa represents a natural or synthetic amino acid residue or a derivative thereof, in the preparation of a medicament for use in the treatment of a neurodegenerative disease or as a cognitive enhancer.
69. A pharmaceutical composition comprising a compound having a formula comprising:

X₁-Arg-Xaa-Arg-X₂ (III)

wherein X₁ and X₂, which may be the same or different, each represents from zero to 32 natural or synthetic amino acid residues or derivatives thereof and Xaa represents a natural or synthetic amino acid residue or a derivative thereof, and a pharmaceutically acceptable carrier.
70. A pharmaceutical composition in which a compound having a formula comprising:

X₁-Arg-Xaa-Arg-X₂ (III)

wherein X₁ and X₂, which may be the same or different, each represents from zero to 32 natural or synthetic amino acid residues or derivatives thereof and Xaa represents a natural or synthetic amino acid residue or a derivative thereof, is chemically or physically linked to a further molecule or vehicle to form a complex for pharmaceutical delivery of said compound.
71. A method of treatment of a neurodegenerative disease in an animal, comprising:
identifying an animal suffering or potentially suffering from a neurodegenerative disease; and
administering to the animal an amount of a compound having a formula comprising:

   X₁-Arg-Xaa-Arg-X₂ (III)
wherein X₁ and X₂, which may be the same or different, each represents from zero to 32 natural or synthetic amino acid residues or derivatives thereof and Xaa represents a natural or synthetic amino acid residue or a derivative thereof, effective to treat the neurodegenerative disease.
72. A method according to clause 70, in which the neurodegenerative disease is Alzheimer's disease.
73. A method of producing a cognitive enhancement effect in an animal, comprising
providing an animal in which the effect is to be produced; and
administering to the animal an amount of a compound having a formula comprising:

X₁-Arg-Xaa-Arg-X₂ (III)

wherein X₁ and X₂, which may be the same or different, each represents from zero to 32 natural or synthetic amino acid residues or derivatives thereof and Xaa represents a natural or synthetic amino acid residue or a derivative thereof, effective to produce the cognitive enhancement.
74. A compound having any of the following formulae:

| | |
|---|---|
| Arg-Glu-Arg | (SEQ ID NO 9) |
| Arg-Glu-Arg-Met | (SEQ ID NO 10) |
| Met-Arg-Glu-Arg | (SEQ ID NO 11) |
| Arg-Glu-Arg-Met-Ser | (SEQ ID NO 3) |
| Ser-Met-Arg-Glu-Arg | (SEQ ID NO 4) |

for use in medicine.
75. Use of a compound having any of the following formulae:

| | |
|---|---|
| Arg-Glu-Arg | (SEQ ID NO 9) |
| Arg-Glu-Arg-Met | (SEQ ID NO 10) |
| Met-Arg-Glu-Arg | (SEQ ID NO 11) |
| Arg-Glu-Arg-Met-Ser | (SEQ ID NO 3) |
| Ser-Met-Arg-Glu-Arg | (SEQ ID NO 4) |

in the preparation of a medicament for use in the treatment of a neurodegenerative disease or as a cognitive enhancer.
76. A pharmaceutical composition comprising a compound having any of the following formulae:

| | |
|---|---|
| Arg-Glu-Arg | (SEQ ID NO 9) |
| Arg-Glu-Arg-Met | (SEQ ID NO 10) |
| Met-Arg-Glu-Arg | (SEQ ID NO 11) |
| Arg-Glu-Arg-Met-Ser | (SEQ ID NO 3) |
| Ser-Met-Arg-Glu-Arg | (SEQ ID NO 4) |

and a pharmaceutically-acceptable carrier.
77. A pharmaceutical composition in which a compound having any of the following formulae:

| | |
|---|---|
| Arg-Glu-Arg | (SEQ ID NO 9) |
| Arg-Glu-Arg-Met | (SEQ ID NO 10) |
| Met-Arg-Glu-Arg | (SEQ ID NO 11) |
| Arg-Glu-Arg-Met-Ser | (SEQ ID NO 3) |
| Ser-Met-Arg-Glu-Arg | (SEQ ID NO 4) |

is chemically or physically linked to a further molecule or vehicle to form a complex for pharmaceutical delivery of said compound.
78. A method of treatment of a neurodegenerative disease in an animal, comprising:
identifying an animal suffering or potentially suffering from a neurodegenerative disease; and
administering to the animal an amount of a compound having any of the following formulae:

| | |
|---|---|
| Arg-Glu-Arg | (SEQ ID NO 9) |
| Arg-Glu-Arg-Met | (SEQ ID NO 10) |
| Met-Arg-Glu-Arg | (SEQ ID NO 11) |
| Arg-Glu-Arg-Met-Ser | (SEQ ID NO 3) |
| Ser-Met-Arg-Glu-Arg | (SEQ ID NO 4) |

effective to treat the neurodegenerative disease.
79. A method according to clause 78, in which the neurodegenerative disease is Alzheimer's disease.
80. A method of producing a cognitive enhancement effect in an animal, comprising
providing an animal in which the effect is to be produced; and
administering to the animal an amount of a compound having any of the following formulae:

| | |
|---|---|
| Arg-Glu-Arg | (SEQ ID NO 9) |
| Arg-Glu-Arg-Met | (SEQ ID NO 10) |
| Met-Arg-Glu-Arg | (SEQ ID NO 11) |
| Arg-Glu-Arg-Met-Ser | (SEQ ID NO 3) |
| Ser-Met-Arg-Glu-Arg | (SEQ ID NO 4) |

effective to produce the cognitive enhancement.

## Claims

1. A pharmaceutical composition comprising: (a) a peptide consisting of any of the following sequences of amino acids:
| | |
|---|---|
| Arg-Glu-Arg | (SEQ ID NO: 9) |
| Arg-Glu-Arg-Met | (SEQ ID NO: 10) |
| Met-Arg-Glu-Arg | (SEQ ID NO: 11) |
| Ser-Met-Arg-Glu-Arg | (SEQ ID NO: 4) |
| Arg-Glu-Arg-Met-Ser | (SEQ ID NO: 3) |
wherein each amino acid residue is in the D-form or the L-form or in the form of a salt thereof, and (b) a pharmaceutically acceptable carrier.

2. A composition according to claim 1, in which the said peptide is chemically or physically linked to a further molecule or vehicle for pharmaceutical delivery of the peptide.

3. A peptide consisting of any of the following sequences of amino acids:
| | |
|---|---|
| Arg-Glu-Arg | (SEQ ID NO: 9) |
| Arg-Glu-Arg-Met | (SEQ ID NO: 10) |
| Met-Arg-Glu-Arg | (SEQ ID NO: 11) |
| Ser-Met-Arg-Glu-Arg | (SEQ ID NO: 4) |
| Arg-Glu-Arg-Met-Ser | (SEQ ID NO: 3) |
wherein each amino acid residue is in the D-form or the L-form or in the form of a salt thereof, for use in medicine.

4. A peptide according to claim 3, which is chemically or physically linked to a further molecule or vehicle for pharmaceutical delivery of the peptide derivative.

5. A peptide according to claim 3 or 4, for use in the treatment of a neurodegenerative disease.

6. A peptide according to claim 3 or 4, for use in the treatment of Alzheimer's disease.

7. Use of a peptide according to claim 3 or 4, in the preparation of a medicament for use in the treatment of a neurodegenerative disease.

8. Use of a peptide according to claim 3 or 4, in the preparation of a medicament for use in the treatment of Alzheimer's disease.

9. A peptide according to claim 3 or 4, for use as a cognitive enhancer.

10. Use of a compound according to claim 3 or 4, in the preparation of a medicament for use as a cognitive enhancer.

11. A method of producing a cognitive enhancement effect in an animal, comprising providing an animal in which the effect is to be produced; and
administering to the animal an amount of a peptide effective to produce the cognitive enhancement,
the peptide having any of the formulae:
| | |
|---|---|
| Arg-Glu-Arg | (SEQ ID NO: 9) |
| Arg-Glu-Arg-Met | (SEQ ID NO: 10) |
| Met-Arg-Glu-Arg | (SEQ ID NO: 11) |
| Ser-Met-Arg-Glu-Arg | (SEQ ID NO: 4) |
| Arg-Glu-Arg-Met-Ser | (SEQ ID NO: 3) |
wherein each amino acid residue is independently in the D-form or the L-form or in the form of a salt thereof.

12. A method of producing a cognitive enhancement effect in an animal, comprising
providing an animal in which the effect is to be produced; and
administering to the animal an amount of a pharmaceutical composition effective to produce the cognitive enhancement,
the pharmaceutical composition comprising a peptide having any of the formulae:
| | |
|---|---|
| Arg-Glu-Arg | (SEQ ID NO: 9) |
| Arg-Glu-Arg-Met | (SEQ ID NO: 10) |
| Met-Arg-Glu-Arg | (SEQ ID NO: 11) |
| Ser-Met-Arg-Glu-Arg | (SEQ ID NO: 4) |
| Arg-Glu-Arg-Met-Ser | (SEQ ID NO: 3) |
wherein each amino acid residue is in the D-form or the L-form or in the form of a salt thereof, and a pharmaceutically acceptable carrier.

13. A method of producing a cognitive enhancement effect in an animal, comprising
providing an animal in which the effect is to be produced; and
administering to the animal an amount of a pharmaceutical composition effective to produce the cognitive enhancement,
the pharmaceutical composition comprising a peptide having any of the formulae:
| | |
|---|---|
| Arg-Glu-Arg | (SEQ ID NO: 9) |
| Arg-Glu-Arg-Met | (SEQ ID NO: 10) |
| Met-Arg-Glu-Arg | (SEQ ID NO: 11) |
| Ser-Met-Arg-Glu-Arg | (SEQ ID NO: 4) |
| Arg-Glu-Arg-Met-Ser | (SEQ ID NO: 3) |
wherein each amino acid residue is in the D-form or the L-form or in the form of a salt thereof, and the at least one compound is chemically or physically linked to a further molecule or vehicle for pharmaceutical delivery of the peptide(s).
